# EUROPÄISCHE PATENTANMELDUNG

(11) **EP 2 650 278 A1**
(43) Veröffentlichungstag der Anmeldung: **16.10.2013**
(21) Anmeldenummer: 12163812.6
(22) Anmeldetag: 11.04.2012
(51) Int. Cl.: C07C 68/06, C07C 68/08, C07C 69/96, C07D 317/36, C07D 317/38, C08G 64/06, C08G 64/30

(54) **Verfahren zur Herstellung von Diarylcarbonaten aus Dialkylcarbonaten**

(71) Anmelder: Bayer MaterialScience AG, 51373 Leverkusen (DE)
(72) Erfinder: Köhler, Karl-Heinz Dr., 52078 Aachen-Brand (DE)
(74) Vertreter: BIP Patents

(57) **Zusammenfassung**

Die vorliegende Erfindung betrifft ein Verfahren zur kontinuierlichen Herstellung von Diarylcarbonaten aus Dialkylcarbonaten und wenigstens einer Monohydroxyverbindung, wobei die Dialkylcarbonatherstellung mehrstufig erfolgt und Alkylenglykol in einer ersten Stufe durch eine oxidative Carbonylierung mit Kohlenmonoxid und Sauerstoff in Gegenwart eines Katalysators zu Alkylencarbonat, welches in einem weiteren Schritt mit einem Alkylalkohol zu Dialkylcarbonat umgesetzt wird, sowie deren Verwendung zur Herstellung von Polycarbonaten. Das bei der Herstellung des eingesetzten Dialkylcarbonats anfallende Alkylenglykol wird durch oxidative Carbonylierung mit Kohlenmonoxid in Gegenwart eines Katalysators in das zyklische Alkylencarbonat rückgefuhrt, das wiederum in das Dialkylcarbonat umgesetzt wird.

## Beschreibung

Die vorliegende Erfindung betrifft ein Verfahren zur kontinuierlichen Herstellung von Diarylcarbonaten aus Dialkylcarbonaten und wenigstens einer Monohydroxyverbindung, wobei die Dialkylcarbonatherstellung mehrstufig erfolgt und Alkylenglykol in einer ersten Stufe durch eine oxidative Carbonylierung mit Kohlenmonoxid und Sauerstoff in Gegenwart eines Katalysators zu Alkylencarbonat, welches in einem weiteren Schritt mit einem Alkylalkohol zu Dialkylcarbonat umgesetzt wird, sowie deren Verwendung zur Herstellung von Polycarbonaten. Das bei der Herstellung des eingesetzten Dialkylcarbonats anfallende Alkylenglykol wird durch oxidative Carbonylierung mit Kohlenmonoxid in Gegenwart eines Katalysators in das zyklische Alkylencarbonat rückgeführt, das wiederum in das Dialkylcarbonat umgesetzt wird.

Es ist bekannt, dass Diarylcarbonate, insbesondere Diphenylcarbonat durch Phasengrenzflächenphosgenierung (Schotten-Baumann-Reaktion) von Monophenolen in einem inerten Lösungsmittel in Gegenwart von Alkali und einem Katalysator hergestellt werden können. Dabei wirkt sich die Verwendung von Lösungsmitteln und Natronlauge nachteilig aus, da durch die wässrige Lauge eine teilweise Verseifung von Phosgen oder Chlorkohlensäureester stattfinden kann, große Mengen Kochsalz als Nebenprodukt anfallen und das Lösungsmittel und Katalysator zurück gewonnen werden müssen.

Auch die Herstellung von aromatischen und aliphatisch-aromatischen Kohlensäurestern (Carbonaten) durch Umesterung ausgehend von aliphatischen Kohlensäurestern und Monophenolen ist im Prinzip bekannt. Dabei handelt es sich um eine Gleichgewichtsreaktion, wobei die Lage des Gleichgewichts fast vollständig in Richtung der aliphatisch substituierten Carbonate verschoben ist. Daher ist es verhältnismäßig leicht, aus aromatischen Carbonaten und Alkoholen aliphatische Carbonate herzustellen. Um die Reaktion jedoch im umgekehrten Sinne in Richtung aromatischer Carbonate durchzuführen, ist es notwendig, das sehr ungünstig liegende Gleichgewicht effektiv auf die Seite der aromatischen Carbonate zu verschieben, wobei nicht nur sehr aktive Katalysatoren, sondern auch geeignete Verfahrensführungen zur Anwendung gelangen müssen.

Die aus der Literatur bekannten Verfahren, wie z.B. EP-A 461 274, DE-A 42 26 755, DE-A 42 26 756, beschreiben jedoch in der Regel nur diejenigen Verfahrensschritte, bei denen die Reaktion zum Diarylcarbonat durch Umesterung und/oder Disproportionierung stattfindet. In WO-A 2006/033291, EP-A 1 775 280, EP-A 1 767 516, EP-A 1 767 517, EP-A 1 767 518, EP-A 1 762 559 und EP-A 1 762 560 werden zudem Hinweise bezüglich der apparativen Ausführungen von Reaktionskolonnen zur Herstellung von Diarylcarbonaten gegeben. Für die Wirtschaftlichkeit eines Verfahrens sind aber nicht nur die Verfahrensabschnitte im Bereich der Reaktion sondern zum Teil in weit größerem Maße die sich anschließenden Schritte für die Aufarbeitung von Relevanz.

Da die Herstellung von Diarylcarbonaten durch Umsetzung einer aromatischen Hydroxyverbindung mit einem Dialkylcarbonat erfahrungsgemäß energetisch sehr aufwändig ist, spielen Maßnahmen zur Reduzierung des Energieverbrauchs ebenfalls eine bedeutende Rolle. Diesbezüglich finden sich in der Literatur auch einige Ansätze zur energetischen und apparativen Integration.

Die Herstellung von Dialkylcarbonaten durch Umesterung von zyklischem Alkylencarbonat und Alkylalkohol ist bekannt und vielfach beschrieben worden. In US-6,930,195 B wurde diese katalysierte Umesterungsreaktion als zweistufige Gleichgewichtsreaktion beschrieben. In der ersten Reaktionsstufe reagiert das zyklische Alkylencarbonat mit Alkylalkohol zu Hydroxyalkylalkylcarbonat als Zwischenprodukt. Das Zwischenprodukt wird dann in der zweiten Reaktionsstufe mit Hilfe von Alkylalkohol umgesetzt zu Dialkylcarbonat und Alkylenglykol.

Für die technische Realisierung des Dialkylcarbonat-Herstellungsverfahrens hat sich die Verwendung einer reaktiven Destillationskolonne (im Folgenden auch Umesterungskolonne genannt), die unter anderem bereits in EP 530 615 A, EP 569 812 A und EP 1 086 940 A beschrieben wurde, als besonders günstig erwiesen. In EP 569 812 A wird das zyklische Alkylencarbonat in den oberen Teil der Umesterungskolonne und der Dialkylcarbonat enthaltende Alkylalkohol in den mittleren oder unteren Teil der Umesterungskolonne kontinuierlich eingeführt. Zusätzlich wird unterhalb der Einführung des Dialkylcarbonat enthaltenden Alkylalkohols nahezu reiner Alkylalkohol eingeführt. Das Schwersiedergemisch, welches das hergestellte Alkylenglykol als Nebenprodukt beinhaltet, wird am Sumpf der Umesterungskolonne kontinuierlich abgezogen. Das Leichtsiedergemisch, welches das hergestellte Dialkylcarbonat beinhaltet, wird am Kopf der Umesterungskolonne als Dialkylcarbonat-Alkylalkohol-Gemisch abgezogen und einem weiteren Aufreinigungsschritt unterworfen.

Nachteilig ist dabei die Bildung des Alkylenglykols als Koppelprodukt, das nur mit guten optischen Eigenschaften (Faserqualität) für die Herstellung von Polyestern verwendet werden kann. Um die guten optischen Eigenschaften zu erreichen, muss daher ein hoher Aufwand zur Reinigung des Alkylenglykols getrieben werden, der die Wirtschaftlichkeit des Verfahrens nachteilig beeinflusst.

Daher wurde auch schon die Umsetzung von Alkylenglykol mit Harnstoff zu zyklischem Alkylencarbonat und Ammoniak in EP 638 541 B1 beschrieben. Nachteilig ist hier, dass Ammoniak für die Polycarbonatherstellung keine Verwendung findet und die Umwandlung von Ammoniak und Kohlendioxid in Harnstoff nicht oder kaum wirtschaftlich ist.

Es ist bekannt, dass die oxidative Carbonylierung von Monoalkoholen mit Kohlenmonoxid und Sauerstoff in Gegenwart von Co- und Cu-Katalysatoren wie CuCl zu Dialkylcarbonat führt wie in EP 463 678 A2 oder EP 413 217 A2 beschrieben. Nachteilig sind dabei die korrosiven Eigenschaften des Katalysatorsystems.

US-A 4,131,521 beschreibt die elektrochemische Oxidation von Dialkoholen wie Ethylenglykol zu Ethylencarbonat. Nachteilig ist dabei die Bildung von Wasserstoff als Nebenprodukt, das in der Polycarbonatherstellung keine Verwendung findet.

Tetrahedron Letters 50, 7330 (2009) beschreibt die Umsetzung von Alkylenglykolen mit Kohlenmonoxid und Sauerstoff mit PdI₂/KI als Katalysatorsystem. Nachteilig ist dabei die geringe Turn Over Number (TON) sowie die geringe Selektivität von 84%.

Die EP 781 760 A1 beschreibt ein kontinuierliches Verfahren zur Herstellung aromatischer Carbonate durch Reaktion eines Dialkylcarbonates mit einer aromatischen Hydroxyverbindung in Anwesenheit eines Katalysators und kontinuierlicher Entfernung des bei der Reaktion entstehenden aromatischen Carbonats, der alkoholischen Nebenprodukte, des Dialkylcarbonats und der aromatischen Hydroxyverbindung, wobei das Dialkylcarbonat und die aromatische Hydroxyverbindung in die Reaktion zurückgeführt werden.

EP 1 638 917 A1 beschreibt ein Verfahren zur Rückgewinnung eines Produktes aus einem Abfallstrom durch Kontaktieren mit einem Alkylalkohol, wobei das rückgewonnene Produkt Diarylcarbonat, aromatischen Alkohol, Alkylsalicylat und Alkylalkohol enthält. Nachteilig am beschriebenen Verfahren ist zum einen, dass die Reaktion in drei Stufen erfolgt, wodurch diese sehr aufwändig wird. Zum anderen fallen an zwei Stellen hochsiedende Abfallströme an. Durch Abtrennung des Katalysators vor der Isolierung des Diarylcarbonats fällt bereits der erste Abfallstrom, bei der sich anschließenden, aus zwei Destillationskolonnen bestehenden Aufarbeitung der zweite Abfallstrom an. Somit ist die Aufarbeitung zur Isolierung des Diarylcarbonats sowohl apparativ als auch energetisch sehr aufwändig. Zudem ist die Qualität des so hergestellten Diarylcarbonats mit 99,5 Gew.-% sehr schlecht und nicht für die Herstellung von Polycarbonat geeignet. Auch die Trennung des bei der Reaktion anfallenden Gemisches aus Reaktionsalkohol und Dialkylcarbonat wird nicht beschrieben.

WO-A 2005/000776 beschreibt ein Verfahren zur Herstellung eines Alkylarylethers, welcher bei der Umsetzung eines Dialkylcarbonats mit einer aromatischen Hydroxyverbindung gebildet wird. Bei diesem Verfahren wird zudem auch Diarylcarbonat erhalten. Der Verfahrensaufbau umfasst drei Reaktionskolonnen und zwei weiteren Destillationskolonnen zwecks Isolierung des Alkylarylethers. Die Tatsache, dass beim hier beschriebenen Verfahren eine gezielte Aufreinigung des Alkylarylethers angestrebt wird lässt darauf schließen, dass die in der Reaktion gebildete Menge hoch ist. Bei der Herstellung von Diarylcarbonaten steht jedoch die Gewinnung eines hochreinen Alkylarylethers nicht im Vordergrund, sonders es ist vielmehr eine möglichst geringe Bildung dieses bei der Umesterung anfallenden Nebenproduktes anzustreben. Zudem ist die Reaktionsführung mit drei Reaktionsstufen sehr aufwändig und bezüglich der Aufarbeitung des Diarylcarbonats und der Trennung des bei der Reaktion anfallenden Gemisches, enthaltend Dialkylcarbonat und Reaktionsalkohol, werden keine Angaben gemacht. Auch in EP-A 1 237 842 A1 wird ein vergleichbares Verfahren beschrieben, weshalb diesbezüglich die bereits genannten Nachteile ebenfalls gelten.

Die WO-A 2004/016577 beschreibt ein Verfahren zur Herstellung von aromatischen Carbonaten aus Dialkylcarbonat und einer aromatischen Hydroxyverbindung in Gegenwart eines Katalysators in mehreren getrennten und in Serie geschalteten Reaktionszonen einer Reaktoranordnung, wobei die anfallende Kondensationswärme bei der Kondensation des Dampfstromes der letzten Reaktionszone zur Erwärmung des in die erste Reaktionszone eingeleiteten Flüssigkeitsstromes verwendet wird. Nachteilig an diesem Verfahren ist jedoch die aufwändige Reaktoranordnung. Zudem ist die energetische Integration dieses Verfahrens verbesserungswürdig und nur auf den Verfahrensabschnitt Reaktion begrenzt. Anschließende Schritte für die Aufarbeitung werden nicht beschrieben.

JP-A 2002/020351 beschreibt ein diskontinuierliches Verfahren zur Herstellung von Diarylcarbonat, aus dem Wärme zur Dampferzeugung genutzt werden kann. Nachteilig an diesem Verfahren sind jedoch die diskontinuierliche Durchführung sowie die zur Umsetzung verwendete Reaktoranordnung mit aufgesetzter Destillationskolonne. Anschließende Schritte für die Aufarbeitung werden nicht beschrieben.

Die chemische Oxidation von Alkoholen mit Kohlenmonoxid und Sauerstoff zu Alkylcarbonaten ist prinzipiell bekannt.

So beschreibt EP 463 678 A2 die oxidative Carbonylierung von Alkoholen mit Kohlenmonoxid und Sauerstoff in Gegenwart von Co- und Cu-Katalysatoren wie CuCl zu Dialkylcarbonat.

Auch die oxidative Carbonylierung von Dialkoholen mit Kohlenmonoxid und Sauerstoff zu Alkylencarbonat ist bekannt.

US-A 4,131,521 beschreibt die Bildung von Ethylencarbonat in 5 bis 10% Ausbeute durch elektrochemische Oxidation von Ethylenglykol in Gegenwart von NH₄Br. Nachteilig ist geringe Wirtschaftlichkeit einer elektrochemischen Oxidation sowie die Bildung von Wasserstoff als Nebenprodukt.

DE A 22 22 488 beschreibt ein Verfahren zur Herstellung von zyklischen Gykolcarbonaten aus Glykolen mit CO/O₂ in Anwesenheit von Kupferionen. Nachteilig ist die geringe Standzeit des Katalysators mit einer TON unter 93 sowie die geringe Aktivität des Katalysators mit einer Turnoverfrequenz unter 30 mol_{Glycol}·mol_{Cu}⁻¹·h⁻¹.

Organometallics, 25, 2 8 7 2 (2006) beschreibt die Herstellung von Ethylencarbonat aus Ethylenglykol Nachteilig ist, dass der teuere Pd-Katalysator in stöchiometrischen Mengen erforderlich ist.

Tetrahedron Lett. 50, 7330 (2009) beschreibt die oxidative Carbonylierung von Dialkoholen wie Ethylenglykol zu Ethylencarbonat mit PdI₂/KI als Katalysatorformulierung. Trotz großem CO-Überschuss wird nur ein mäßiger TON erwähnt.

J. Org. Chem. 51. 2977 (1986) beschreibt die Umsetzung von Ethylenglycol und 1-Phenylethandiol zu den entsprechenden zyklischen Carbonaten. Nachteilig ist der Einsatz von Metallsalzen als Oxidanz in stöchiometrischen Mengen.

EP 1 125 915 B 1 beschreibt die Reaktion von Ethylenoxid mit CO₂ in Gegenwart eines Katalysators zu zyklischen Carbonaten, sowie die anschließende Umesterung mit Alkoholen zu Dialkylcarbonaten. Nachteilig ist der Einsatz von Ethylenoxid als Startmaterial, das, wie dem Fachmann bekannt ist, schwer zu handhaben ist.

Es bestand demnach Bedarf, ein Verfahren zur Herstellung von aromatischen Carbonaten, vorzugsweise Diarylcarbonaten, sowie Polycarbonate, welches Produkte in hoher Reinheit und guter Ausbeute liefert und eine Reduzierung der Umweltbelastung durch maximierte Rückführung von Nebenprodukten, die aus der Diarylcarbonat- und Polycarbonat-Produktion stammen, erreicht wird.

Die Aufgabe, die der Erfindung zugrunde lag, bestand demnach darin, ein Verfahren zur Herstellung von aromatischen Carbonaten, vorzugsweise Diarylcarbonaten, bereitzustellen, welches

Die Aufgabe wurde dadurch gelöst, dass eine maximierte Rückführung des zwangsanfallenden Alkylenglykols ermöglicht wird, eine Notwendigkeit einer aufwändigen Aufreinigung bei der Rückführung der Edukte vermieden wird, wodurch als Edukte weitestgehend nur Kohlenmonoxid, Sauerstoff und Bisphenol verwendet werden.

Gegenstand der vorliegenden Erfindung daher ist ein Verfahren zur Herstellung von Diarylcarbonaten und Polycarbonaten, ausgehend von Dialkylcarbonaten, die aus der Umsetzung von Alkylencarbonat mit einem Alkylalkohol, und Monophenolen, das Alkylencarbonat wiederrum durch oxidative Carbonylierung von Alkylenglycol als Edukt erhalten wird, das dadurch gekennzeichnet ist, dass das bei der Herstellung des eingesetzten Dialkylcarbonats aus zyklischem Alkylencarbonat und Alkylalkohol entstehende Alkylenglykol durch Reaktion mit Kohlenmonoxid zum Alkylencarbonat rückgeführt wird, welches anschließend wieder mit dem Alkylalkohol zur Herstellung des Dialkylcarbonats eingesetzt wird. Das Monophenol, das bei der Herstellung von lösungsmittelfreiem Polycarbonat durch Umesterung von Diarylcarbonaten und Bisphenolen freigesetzt wird, kann wiederum zur Herstellung des Diarylcarbonats verwendet werden.

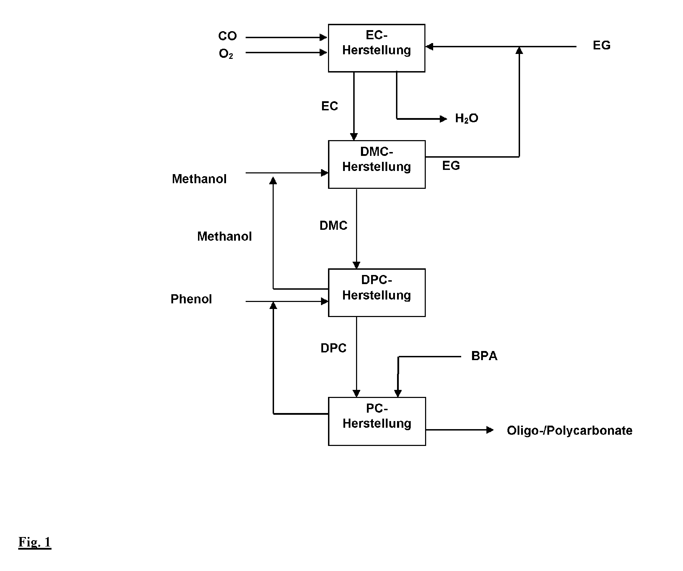

Das erfindungsgemäße Gesamtverfahren ist flexibel, einfach in der Durchführung und liefert Produkte in hoher Reinheit, die für den Gesamtprozess außerordentlich wichtig sind unter gleichzeitiger Reduzierung der Umweltbelastung durch Wiederverwendung von Edukten aus dem Prozess, umfassend folgende Prozeßschritte (vgl. Fig. 1):
(a) Herstellung von Alkylencarbonat durch oxidative Carbonylierung von Alkylenglycol mit Kohlenmonoxid und Sauerstoff unter Bildung von Wasser,
(b) Abtrennung und gegebenenfalls Reinigung wenigstens eines Teils des in Schritt (a) gebildeten Alkylencarbonats,
(c) Umsetzung des gemäß Schritt (b) abgetrennten Alkylencarbonats mit wenigstens einem Alkylalkohol in Gegenwart eines Katalysators, und gegebenenfalls organischem Lösungsmittel unter Bildung wenigstens eines Dialkylcarbonats und von Alkylenglykol,
(d) Abtrennung und Aufarbeitung wenigstens eines Teils des in Schritt (c) gebildeten Dialkylcarbonats,
(e) Abtrennung und gegebenenfalls Reinigung wenigstens eines Teils des gemäß Schritt (c) gebildeten Alkylenglykols,
(f) Rückführung wenigstens eines Teils des gemäß Schritt (e) abgetrennten Alkylenglykols, gegebenenfalls enthaltend Dialkylcarbonat(e), zum Schritt (a),
(g) Umsetzung wenigstens eines Teils des gemäß Schritt (e) abgetrennten Dialkylcarbonats mit einem Monophenol zum Alkylarylcarbonat und/oder Diarylcarbonat und Alkylalkohol; das Alkylalkohol kann optional wiederum in Schritt (c) eingesetzt werden,
(h) Disproportionierung wenigstens eines Teils des gemäß Schritt (g) hergestellten Alkylarylcarbonats zum Diarylcarbonat und Dialkylcarbonat, das wiederum in Schritt (c) eingesetzt werden kann,
(i) Umesterung wenigstens eines Teils des gemäß Schritt (h) hergestellten Diarylcarbonats mit einem Bisphenol zum Oligo-/Polycarbonat und dem Monophenol; das Monophenol kann wiederum in Schritt (g) eingesetzt werden.

Im Rahmen der Erfindung verwendete zyklische Alkylencarbonate wie sie in Schritt (a) hergestellt und in Schritt (c) eingesetzt werden sind bevorzugt solche mit der Formel (I): 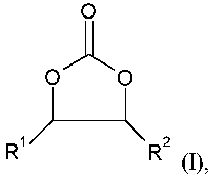 wobei in der Formel (I) R¹ und R² unabhängig voneinander Wasserstoff, substituiertes oder nicht substituiertes **C₁-C₄-Alkyl**, substituiertes oder nicht substituiertes **C₂-C₄-Alkenyl** oder substituiertes oder nicht substituiertes **C₆-C₁₂-Aryl** und R¹ und R² gemeinsam mit den beiden Fünfring-C-Atomen einen gesättigten carbozyklischen Ring mit 5 - 8 Ringgliedern bedeuten können. Bevorzugt sind Ethylencarbonat, Propylencarbonat und Butylencarbonat, besonders bevorzugt Ethylencarbonat und Propylencarbonat, ganz besonders bevorzugt Ethylencarbonat.

Die zyklischen Alkylencarbonate werden mit Alkoholen der Form

R³-OH

zu Dialkylcarbonaten umgesetzt.

Bei dieser Umsetzung entsteht ein Alkylenglycol der Formel R²(OH)₂ als Nebenprodukt, das in Schritt (e) vom Hauptprodukt Dialkylcarbonat abgetrennt und gegebenenfalls aufgereinigt wird.

Im Rahmen der Erfindung bevorzugt eingesetzte Dialkylcarbonate sind solche der Formel (II)

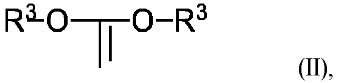

R³ steht für lineares oder verzweigtes, gegebenenfalls substituiertes C₁-C₃₄-Alkyl, bevorzugt für C₁-C₆-Alkyl, besonders bevorzugt für C₁-C₄-Alkyl.

C₁-C₄-Alkyl in der Formel (II) steht beispielsweise für Methyl, Ethyl, n-Propyl, iso-Propyl, n-Butyl, sec.-Butyl, tert.-Butyl, C₁-C₆-Alkyl darüber hinaus beispielsweise für n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, C₁-C₃₄-Alkyl darüber hinaus beispielsweise für n-Heptyl und n-Octyl, Pinakyl, Adamantyl, die isomeren Menthyle, n-Nonyl, n-Decyl, n-Dodecyl, n-Tridecyl, n-Tetradecyl, n-Hexadecyl oder n-Octadecyl. Gleiches gilt für den entsprechenden Alkylrest beispielsweise in Aralkyl- bzw. Alkylarylresten. Alkylenreste in den entsprechenden Hydroxyalkyl- oder Aralkyl- bzw. Alkylarylresten stehen beispielsweise für die den vorangehenden Alkylresten entsprechenden Alkylenreste.

Aryl steht für einen carbozyklischen aromatischen Rest mit 6 bis 34 Gerüstkohlenstoffatomen. Gleiches gilt für den aromatischen Teil eines Arylalkylrestes, auch Aralkylrest genannt, sowie für Arylbestandteile komplexerer Gruppen, wie z.B. Arylcarbonylresten.

Arylalkyl bzw. Aralkyl bedeutet jeweils unabhängig einen geradkettigen, zyklischen, verzweigten oder unverzweigten Alkyl-Rest nach vorstehender Definition, der einfach, mehrfach oder vollständig durch Aryl-Reste gemäß vorstehender Definition substituiert sein kann.

Die vorangehenden Aufzählungen sind beispielhaft und nicht als Limitierung zu verstehen.

Bevorzugte Dialkylcarbonate sind Dimethylcarbonat, Diethylcarbonat, Di(n-propyl)carbonat, Di(iso-propyl)carbonat, Di(n-butyl)carbonat, Di(sec-butyl)carbonat, Di(tert-butyl)carbonat oder Dihexylcarbonat. Besonders bevorzugt sind Dimethylcarbonat oder Diethylcarbonat. Ganz besonders bevorzugt ist Dimethylcarbonat.

Die einsetzbaren Umesterungskatalysatoren für die Herstellung von Dialkylcarbonaten in Schritt (c) aus dem zyklischen Alkylencarbonat aus Schritt (a) und dem Alkohol sind die dem Fachmann bekannten, beispielsweise Hydride, Oxide, Hydroxide, Alkoholate, Amide oder Salze von Alkalimetallen, wie Lithium, Natrium, Kalium, Rubidium und Cäsium, bevorzugt von Lithium, Natrium und Kalium, besonders bevorzugt von Natrium und Kalium (US 3,642,858 A, US 3 803 201 A, EP 1 082 A). Für den Fall des Einsatzes der Alkoholate können diese erfindungsgemäß auch in situ durch Einsatz der elementaren Alkalimetalle und dem erfindungsgemäßen umzusetzenden Alkohol gebildet werden. Salze der Alkalimetalle können solche von organischen oder anorganischen Säuren sein, wie von Essigsäure, Propionsäure, Buttersäure, Benzoesäure, Stearinsäure, Kohlensäure (Carbonate oder Hydrogencarbonate), von Salzsäure, Bromwasserstoff- oder lodwasserstoffsäure, Salpetersäure, Schwefelsäure, Fluorwasserstoffsäure, Phosphorsäure, Blausäure, Rhodanwasserstoff, Borsäure, Zinnsäure, C₁-C₄-Stannonsäuren oder Antimonsäuren. In bevorzugter Weise kommen als Verbindungen der Alkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt. Solche Alkalimetallverbindungen (gegebenenfalls in situ gebildet aus den freien Alkalimetallen) werden in Mengen von 0,001 bis 2 Gew.-%, bevorzugt 0,003 bis 1,0 Gew.-%, besonders bevorzugt 0,005 bis 1,0 Gew.-%, bezogen auf das umzusetzende Reaktionsgemisch, eingesetzt.

Als Katalysatoren für das Verfahren in Schritt (c) sind ferner Thallium-I- und Thallium-III-Verbindungen geeignet, wie die Oxide, Hydroxide, Carbonate, Acetate, Bromide, Chloride, Fluoride, Formiate, Nitrate, Cyanate, Stearate, Naphthenate, Benzoate, Cyclohexylphosphonate, Hexahydrobenzoate, das Cyclopentadienylthallium, Thalliummethylat, Thalliumethylat, bevorzugt Tl-(I)-oxid, Tl-(I)-hydroxid, Tl-(I)-carbonat, Tl-(I)-acetat, Tl-(III)-acetat, Tl-(I)-fluorid, Tl-(I)-formiat, Tl-(I)-nitrat, Tl-(I)-naphtenat und Tl-(I)-methylat (EP 1 083). Die Mengen an Thalliumkatalysator sind nicht besonders kritisch. Sie betragen im allgemeinen 0,0001-10 Gew.-%, bevorzugt 0,001-1 Gew.-%, bezogen auf das gesamte Reaktionsgemisch. Im erfindungsgemäßen Verfahren können weiterhin stickstoffhaltige Basen als Katalysatoren eingesetzt werden (US 4 062 884). Genannt seien beispielsweise *sek.* oder *tert.* Amine wie Triethylamin, Tributylamin, Methyldibenzylamin, Dimethylcyclohexylamin u.a..

Die eingesetzten Mengen der stickstoffhaltigen Basen in Schritt (c) liegen von 0,01 bis 10 Gew.-%, bevorzugt von 0,1 bis 5 Gew.-%, besonders bevorzugt von 0,1 bis 1 Gew.-%, bezogen auf das gesamte Reaktionsgemisch. Als Katalysatoren sind erfindungsgemäß ferner Verbindungen aus der Gruppe der Phosphine, Stibine, Arsine oder der zweiwertigen Schwefel- und Selen-Verbindungen sowie deren Oniumsalze einsetzbar (EP 180 387, US 4 734 519).

Beispielhaft seien die folgenden genannt: Tributylphosphin, Triphenylphosphin, Diphenylphosphin, 1,3-Bis-(diphenylphosphino)propane, Triphenylarsin, Trimethylarsin, Tributylarsin, 1,2-Bis-(diphenylarsino)ethan, Triphenylantimon, Diphenylsulfid, Diphenyldisulfid, Diphenylselenid, Tetraphenylphosphoniumhalogenid (CI, Br, J), Tetraphenylarsoniumhalogenid (CI, Br, J), Triphenylsulfoniumhalogenid (Cl, Br) usw.

Die Einsatzmengen dieser Katalysatorengruppe liegen im Bereich von 0,1 bis 10 Gew.-%, bevorzugt von 0,1 bis 5 Gew.-%, besonders bevorzugt im Bereich von 0,1 bis 2 Gew.-%, bezogen auf das gesamte Reaktionsgemisch.

Weiterhin einsetzbar sind Komplexe oder Salze des Zinns, Titans oder Zirkoniums. Beispiele solcher Systeme sind Butylstannonsäure, Zinnmethoxid, Dimethylzinn, Dibutylzinnoxid, Dibutylzinndilaurat, Tributylzinnhydrid, Tributylzinnchlorid, Zinn(II)ethylhexanoate, Zirkoniumalkoxide (Methyl, Ethyl, Butyl), Zirkonium(IV)halogenide (F, CI, Br, J), Zirkoniumnitrate, Zirkoniumacetylacetonat, Titanalkoxide (Methyl, Ethyl, Isopropyl), Titanacetat, Titanacetylacetonat usw.

Die erfindungsgemäß einsetzbaren Mengen betragen 0,1 bis 10 Gew.-%, bevorzugt 0,1 bis 5 Gew.-%, bezogen auf das Gesamtgemisch.

Im vorliegenden Verfahren können weiterhin in Schritt (c) bifunktionelle Katalysatoren der Formel (III)

[AₐX_{b}]ₘ • [B_{c}Y_{d}]ₙ (III)

eingesetzt werden. In diesen bifunktionellen Katalysatoren wird das molare Verhältnis der beiden in eckigen Klammern stehenden Komponenten durch die Indices m und n ausgedrückt. Diese Indices können unabhängig voneinander Werte von 0,001-1, bevorzugt 0,01-1, besonders bevorzugt 0,05-1 und ganz besonders bevorzugt 0,1-1 annehmen. Innerhalb der eckigen Klammern stehen neutrale Salze aus je einem Kation und einem Anion. Die Indices a und b stellen unabhängig voneinander ganze Zahlen von 1-5 dar; die Indices C und d bedeuten unabhängig voneinander ganze Zahlen von 1-3, wobei den Forderungen der Valenzen der Kationen und Anionen zur Bildung solcher neutraler Salze zu entsprechen ist. Des weiteren bedeuten in (VI) A das Kation eines Metalles, das der dritten Periode und Gruppe IIa, der vierten Periode und Gruppe IIa, IVa-VIIIa, Ib oder IIb, der fünften Periode und Gruppe IIa, IVa-VIIa oder IVb bzw. der sechsten Periode und Gruppe IIa-VIa des Periodensystems der Elemente in der Kurzperiodenform angehört.

Die in Frage kommenden Metalle für das Kation A entnimmt der Fachmann den üblichen Darstellungen des Periodensystems der Elemente (Mendelejew) in der Kurzperiodenform. In bevorzugter Weise handelt es sich bei A um das Kation eines der Metalle Mg, Ca, Sr, Ba, Zn, Cu, Mn, Co, Ni, Fe, Cr, Mo, W, Ti, Zr, Sn, Hf, V und Ta, in bevorzugter Weise um das Kation eines der Metalle Mg, Ca, Zn, Co, Ni, Mn, Cu und Sn. Außer den nicht komplexierten Kationen der genannten Metalle kommen auch kationische Oxokomplexe der genannten Metalle in Frage, wie beispielsweise Titanyl TiO⁺⁺ und Chromyl CrO₂⁺⁺.

Das zum Kation A gehörige Anion X ist das einer anorganischen oder organischen Säure. Eine solche anorganische oder organische Säure kann einbasisch oder zweibasisch oder dreibasisch sein. Solche Säuren und ihre Anionen sind dem Fachmann bekannt. Beispiele für Anionen einbasischer anorganischer oder organischer Säuren sind: Fluorid, Bromid, Chlorid, lodid, Nitrat, das Anion einer Alkancarbonsäure mit 1-18 C-Atomen und Benzoat; Beispiele für Anionen zweibasischer anorganischer oder organischer Säuren sind: Sulfat, Oxalat, Succinat, Fumarat, Maleinat, Phthalat und weitere; Beispiele für dreibasische anorganische oder organische Anionen sind: Phosphat oder Citrat. Bevorzugte Anionen X im Katalysator der Formel (III) sind: Fluorid, Chlorid, Bromid, lodid, Sulfat, Nitrat, Phosphat, Formiat, Acetat, Propionat, Oxalat, Butyrat, Citrat, Succinat, Fumarat, Maleinat, Benzoat, Phthalat, Decanoat, Stearat, Palmitat, und Laurinat. Besonders bevorzugte Anionen X sind: Chlorid, Bromid, lodid, Acetat, Laurinat, Stearat, Palmitat, Decanoat, Nitrat und Sulfat.

Als Kation B in den Katalysatoren der Formel (III) kommt eines aus der Gruppe der Alkali- oder Erdalkalikationen, der quaternären Ammonium-, Phosphonium-, Arsonium- oder Stibonium-Kationen und der ternären Sulfonium-Kationen in Frage.

Als (Erd)Alkalimetallkationen seien hierbei genannt: das Lithium-, Natrium-, Kalium-, Rubidium-, Cäsium-, Magnesium-, Calcium-, Strontium- und Bariumkation, bevorzugt die genannten Alkalimetallkationen, besonders bevorzugt das Natrium- und das Kaliumkation.

In bevorzugter Weise kommen als Kationen B solche der Formel (IV) 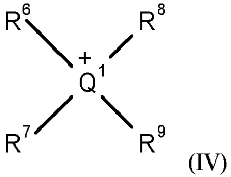 in Frage, worin
Q¹ für N, P, As oder Sb steht und
R⁶, R⁷, R⁸ und R⁹ unabhängig voneinander geradkettiges oder verzweigtes C₁-C₁₈ oder C₇-C₁₂-Aralkyl sind und einer der Reste R⁶-R⁹ auch sein kann. In besonders bevorzugter Weise ist B ein Kation der Formel (V) 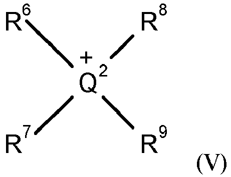 worin,
Q² für N oder P, bevorzugt für N steht.

In ganz besonders bevorzugter Weise treten im Rahmen der Formeln (IV) bzw. (V) an die Stelle der Reste R⁶, R⁷, R⁸ und R⁹ die Reste R¹⁶, R¹⁷, R¹⁸ bzw. R¹⁹, die unabhängig voneinander geradkettiges oder verzweigtes C₁-C₁₈-Alkyl oder C₇-C₈-Aralkyl bedeuten und einer der Reste R¹⁶ bis R¹⁹ auch Phenyl sein kann. In weiterhin ganz besonders bevorzugter Weise treten an die Stelle der Reste R¹⁶, R¹⁷, R¹⁸ bzw. R¹⁹, die Reste R²⁶, R²⁷, R²⁸ bzw. R²⁹, die unabhängig voneinander C₁-C₈-Alkyl oder Benzyl bedeuten und einer der Reste R²⁶ bis R²⁹ auch Phenyl sein kann.

Geradkettiges oder verzweigtes C₁-C₁₈-Alky ist beispielsweise Methyl, Ethyl, Propyl, Isopropyl, Butyl, Isobutyl, Hexyl, Octyl, Dodecyl, Hexadecyl oder Octadecyl. Bevorzugtes Alkyl hat 1-12 C-Atome, besonders bevorzugtes Alkyl hat 1-8 C-Atome.

C₇-C₁₂-Aralkyl ist beispielsweise Benzyl, Phenylethyl, Phenylpropyl, Naphthylmethyl oder Naphthyl-ethyl; bevorzugtes Aralkyl ist Benzyl oder Phenylethyl, ganz besonders bevorzugtes Aralkyl ist Benzyl.

C₆-C₁₂-Aryl ist beispielsweise Phenyl, Naphthyl oder Biphenylyl, bevorzugt Phenyl.

Das Anion Y im Katalysator der Formel (IV) ist ein Halogenidion, wie Fluorid, Chlorid, Bromid oder lodid, bevorzugt Bromid oder lodid, besonders bevorzugt lodid. Es kann jedoch auch die Bedeutung von anderen unter X genannten Anionen besitzen, wenn im konkreten Fall das Anion X Bromid oder lodid ist.

Der bifunktionelle Katalysator der Formel (IV) wird in einer Menge von 0,005-5 Gew.-%, bevorzugt 0,01-3 Gew.-%, besonders bevorzugt 0,01-1 Gew.-%, bezogen auf das gesamte Umesterungsgemisch, eingesetzt.

Das Verfahren zur Herstellung von Dialkylcarbonat aus Alkylencarbonat und Alkylalkohol kann kontinuierlich oder diskontinuierlich durchgeführt werden. Bevorzugt ist eine kontinuierliche Fahrweise.

Die Reaktion von Alkylalkohol und Alkylencarbonat zu Dialkylcarbonat und Alkylenglykol kann in verschiedenartigen Apparaten Kolonnen, Rohrreaktoren, Reaktoren mit aufgesetzten Kolonnen usw. im Gleich- oder Gegenstrom durchgeführt werden. Bevorzugt wird die Reaktion in einer oder mehreren Kolonnen im Gegenstrom, im Folgenden. Umesterungskolonne genannt, durchgeführt.

Im Verfahren werden die zyklische(n) Alkylencarbonatverbindung(en) und der oder die Alkylalkohol(e) bevorzugt im Molverhältnis von 1 : 0.1 bis 1 : 40, besonders bevorzugt von 1 : 1.0 bis 1 : 30, ganz besonders bevorzugt von 1 : 2.0 bis 1 : 20 eingesetzt. Dabei berücksichtigt das angegebene Molverhältnis nicht die Rückführung von zyklischer Alkylencarbonatverbindung oder Alkohol in die Umesterungskolonne über einen oder mehrere Kopfkondensator(en) oder einen oder mehrere etwaig vorhandene(n) Sumpfverdampfer.

Der Katalysator wird bevorzugt zusammen mit dem Strom enthaltend das zyklische Alkylencarbonat in gelöster oder suspendierter Form in die Umesterungskolonne über eine Einführungsstelle, die oberhalb der Einführungsstellen des Alkylalkohols angeordnet ist, in die Kolonne eingebracht. Alternativ kann der Katalysator auch separat beispielsweise im Alkylalkohol, im Alkylenglykol oder in einem geeigneten inerten Lösungsmittel gelöst, zudosiert werden. Im Falle der Verwendung heterogener Katalysatoren können diese im Gemisch mit Füllkörpern, in geeigneter Form anstelle von Füllkörpern oder als Schüttung auf etwaig eingebaute Kolonnenböden eingesetzt werden.

Der Umsatz von Alkylencarbonat und Alkylalkohol zu Dialkylcarbonat und Alkylenglykol findet fast vollständig in der Umesterungskolonne statt. In bevorzugten Ausführungsformen des Verfahrens zur Herstellung von Dialkylcarbonat kann der am Sumpf dieser Umesterungskolonne entnommene Flüssigkeitsstrom - gegebenenfalls nach Aufkonzentrierung - in einem oder mehreren weiteren Schritten einer weiteren Reaktion und/oder Reinigung unterzogen werden. Bevorzugt können einzelne oder alle solche weiteren Schritte in einer oder mehreren weiteren Kolonnen oder in anderen zur Reinigung geeigneten Apparaten wie Fallfilmverdampfern oder Dünnschichtverdampfern erfolgen.

Als Umesterungskolonne oder gegebenenfalls zweite bzw. weitere Kolonne(n) kommen dem Fachmann bekannte Kolonnen in Frage. Dies sind beispielsweise Destillations- bzw. Rektifikationskolonnen, bevorzugt Reaktivdestillations- bzw. Reaktivrektifikationskolonnen.

Die Umesterungskolonne enthält bevorzugt wenigstens einen Verstärkungsteil im oberen Teil der Kolonne und wenigstens eine Reaktionszone unterhalb des Verstärkungsteils. Jede der zwei Sektionen weist unabhängig voneinander bevorzugt jeweils 0 bis 30, bevorzugt 0,1 bis 30 theoretische Stufen auf.

In bevorzugten Ausführungsformen weist die Umesterungskolonne unterhalb einer Reaktionszone wenigstens einen Abtriebsteil auf.

Die Umesterungskolonne kann weiterhin bevorzugt mit einem oder mehreren Sumpfverdampfer(n) ausgestattet sein. Bei Ausführung der Umesterungskolonne mit einem Abtriebsteil wird bevorzugt zusätzlich ein Sumpfverdampfer eingesetzt, welcher die aus dem Abtriebsteil ablaufende Flüssigkeit ganz oder teilweise verdampft. Dieser ganz oder teilweise verdampfte Flüssigkeitsstrom wird ganz oder teilweise wieder in die Umesterungskolonne zurückgeführt. Im Falle einer Ausführungsform ohne Abtriebsteil wird in einem gegebenenfalls eingesetzten Sumpfverdampfer die aus der Reaktionszone ablaufende Flüssigkeit ganz oder teilweise verdampft und ganz oder teilweise wieder in die Umesterungskolonne zurückgeführt.

Der oder die Verstärkungsteil(e) können in bevorzugten Ausführungsformen zusammen mit dem oder den Reaktionsteil(en) und gegebenenfalls wenigstens einem Abtriebsteil in der Umesterungskolonne untergebracht werden. Dabei wird das aus der oder den Reaktionszone(n) kommende dampfförmige Gemisch von unten in eine untere Sektion des Verstärkungsteils bzw. gegebenenfalls den unteren Verstärkungsteil geleitet, wobei eine Abreicherung des Alkylencarbonats bzw. Alkylenglykols stattfindet.

Unterhalb der Reaktionszone und eines gegebenenfalls vorhandenen Abtriebsteils wird ein Gemisch enthaltend Alkylenglykol, überschüssigem oder nicht umgesetztem Alkylencarbonat, Alkylalkohol, Dialkylcarbonat, Umesterungskatalysatoren und bei der Reaktion entstehende oder bereits in den Edukten enthaltene schwersiedende Verbindungen erhalten. Bei Verwendung eines Abtriebsteils wird der Gehalt an leichtsiedenden Verbindungen, wie beispielsweise Dialkylcarbonat und Alkohol reduziert, wobei in Anwesenheit des Umesterungskatalysators unter Umständen weiteres Dialkylcarbonat und Alkylenglykol aber auch Dialkylenglykol und weitere hochsiedende Verbindungen gebildet werden. Die hierzu erforderliche Energie, wird bevorzugt durch einen oder mehrere Verdampfer zugeführt.

In allen Abschnitten der Umesterungskolonne, d.h. sowohl in Verstärkungs- und gegebenenfalls Abtriebsteil als auch in der Reaktionszone können Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl" Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novolaxringe, Berlsättel, BX-Packungen, Montz Pak, Mellapak, Melladur, Kerapak und CY-Packungen.

Alternativ eignen sich auch Kolonnenböden, wie beispielesweise Sieb-, Glocken-, Ventil-, Tunnelböden. In der oder den Reaktionszone(n) der Umesterungskolonne sind Kolonnenböden mit hohen Verweilzeiten bei gutem Stoffaustausch, beispielsweise Glockenböden, Ventil- oder Tunnelböden mit hohen Überlaufwehren, besonders bevorzugt. Die theoretische Bodenzahl der Reaktionszone beträgt vorzugsweise 3 bis 50, besonders bevorzugt 10 bis 50 und ganz besonders bevorzugt 10 bis 40. Der Flüssigkeits-Hold-up beträgt vorzugsweise 1 bis 80%, besonders bevorzugt 5 bis 70% und ganz besonders bevorzugt 7 bis 60% des Kolonneninnenvolumens der Reaktionszone. Die genauere Auslegung der Reaktionszone(n), des gegebenenfalls zu verwendendes Abtriebsteils und des oder der Verstärkerteil(e) kann vom Fachmann vorgenommen werden.

Die Temperatur der Reaktionszone(n) liegt bevorzugt im Bereich von 20 bis 200°C, besonders bevorzugt von 40 bis 180°C, ganz besonders bevorzugt von 50 bis 160°C. Es ist von Vorteil, die erfindungsgemäße Umesterung nicht nur bei Normaldruck, sondern auch bei erhöhtem oder erniedrigtem Druck durchzuführen. Der Druck der Reaktionszone liegt daher bevorzugt im Bereich von 0,2 bis 20 bar, besonders bevorzugt von 0,3 bis 10 bar, ganz besonders bevorzugt von 0,4 bis 5 bar. Bei den vorangehend und im Folgenden aufgeführten Druckangaben handelt es sich - sofern nichts anderes explizit erwähnt - um abolute Druckangaben.

Bevorzugt wird das am Kopf der Umesterungskolonne in dem Verfahren zur Herstellung des Dialkylcarbonates entnommene Dampfgemisch enthaltend Dialkylcarbonat und Alkylalkohol nach Kondensation am Kopf der Umesterungskolonne ganz oder teilweise wenigstens einem weiteren Verfahrensschritt enthaltend wenigstens eine Destillationskolonne zur Trennung von Dialkylcarbonat und Alkylalkohol zugeführt.

Die Trennung des Dialkylcarbonats und des Alkylalkohols erfolgt bevorzugt destillativ in einer oder mehreren Destillationskolonnen oder in einer Kombination aus Destillation und Membrantrennung - im Folgenden als Hybridprozess - bezeichnet (siehe z. B. US-4,162,200 A, EP 581 115 B1, EP 592 883 B1 und WO 2007/096343A1).

Bilden Alkylalkohol und Dialkylcarbonat ein Azeotrop (z.B. Methanol und Dimethylcarbonat) so kann auch ein zweistufiges Verfahren wie beispielsweise ein Zweidruckverfahren, eine Extraktivdestillation, eine Heteroazeotropdestillation mit einem niedrigsiedenden Schleppmittel oder ein Hybridverfahren verwendet werden. Besonders bevorzugt wird das Zweidruckverfahren oder ein Hybridverfahren angewendet.

Ganz besonders bevorzugt wird die Trennung des Dialkylcarbonats und des Alkylalkohols - selbst in dem Falle, dass das Dialkylcarbonat und der Alkylalkohol ein Azeotrop bilden - in einer einzelnen Destillationskolonne durchgeführt. Diese Destillationskolonne wird bei einem Druck betrieben, der höher ist als der Druck der Umesterungskolonne(n). Der Betriebsdruck der Destillationskolonne liegt im Bereich von 1 bis 50 bar, vorzugsweise zwischen 2 und 20 bar Am Sumpf der Destillationskolonne wird das nahezu reine Dialkylcarbonat entnommen und am Kopf ein Gemisch aus Dialkylcarbonat und Alkylalkohol. Dieses Gemisch wird der oder den Umesterungskolonne(n) ganz oder teilweise zugeführt. Wird das Verfahren zur Herstellung von Dialkylcarbonat mit einem Verfahren zur Herstellung von Diarylcarbonat, welches durch Umesterung dieses Dialkylcarbonates mit einer aromatischen Hydroxyverbindung gebildet wird, gekoppelt, so kann ein Teil des Gemisches aus Dialkylcarbonat und Alkylalkohol, welches am Kopf der Destillationskolonne entnommen wird, einem entsprechenden Aufarbeitungsschritt für Alkylalkohol und Dialkylcarbonat in der Verfahrensstufe zur Herstellung von Diarylcarbonat zugeführt werden.

In einer besonders bevorzugten Ausführung, wenn das Dialkylcarbonat und der Alkylalkohol ein Azeotrop bilden, ist dieser Aufarbeitungsschritt ein Zweidruckverfahren. Solche Verfahren sind dem Fachmann grundsätzlich bekannt (vgl. z.B. Ullmann's Encyclopedia of Industrial Chemistry, 6th edition, Vol. 10, Kap. 6.4. und 6.5.; Chemie Ingenieur Technik (67) 11 / 95).

Bilden Alkylalkohol und Dialkylcarbonat ein Azeotrop, so weist das Destillat einer ersten Destillationskolonne des Verfahrensschritts zur Trennung von Dialkylcarbonat und Alkylalkohol vorzugsweise nahezu azeotrope Zusammensetzung auf. In diesem Fall wird dieses bevorzugt in einem Zweidruckverfahren wenigstens einer weiteren Destillationskolonne zugeführt die bei einem Betriebsdruck arbeitet, der unter dem der ersten Destillationskolonne liegt. Durch den unterschiedlichen Betriebsdruck verschiebt sich die Lage des Azeotrops hin zu geringeren Anteilen an Alkylalkohol. Man erhält als Sumpfprodukt dieser zweiten oder weiteren Destillationskolonne(n) Alkylalkohol in einer Reinheit von 90 bis 100 Gew.-%, bezogen auf das Gesamtgewicht des isolierten Sumpfproduktes, und als Destillat eine nahezu azeotropes Gemisch. Die bei geringerem Betriebsdruck arbeitende zweite oder weitere(n) Destillationskolonne(n) wird in ganz besonders bevorzugten Ausführungsformen vorzugsweise mit der Kondensationswärme des oder der Kopfkondensator(en) der ersten Destillationskolonne betrieben.

Der Betriebsdruck der zweiten Destillationskolonne, Alkylalkoholkolonne genannt, wird vorzugsweise so gewählt, dass man diese mit der Abwärme der Dialkylcarbonatkolonne betreiben kann. Der Betriebsdruck liegt dabei zwischen 0,1 und 1 bar vorzugsweise zwischen 0,3 und 1 bar. Der Betriebsdruck der Dialkylcarbonatkolonne liegt im Bereich von 1 bis 50 bar, vorzugsweise zwischen 2 und 20 bar.

Am Sumpf der Umesterungskolonne wird ein Gemisch entnommen, das im wesentlichen Alkylenglykol und in geringen Mengen Alkylalkohol, Dialkylcarbonat, Alkylencarbonat und Hochsieder (wie z.B. Dialkylenglykol) enthält. Wird ein homogener Katalysator verwendet, so ist dieser ebenfalls in diesem Gemisch enthalten. Dieses Gemisch wird einer weiteren Aufarbeitung zugeführt, um Alkylenglykol aufzureinigen, unerwünschte Komponenten weitgehend aus dem Prozess auzuschleusen und den gegebenenfalls verwendeten homogenen Katalysator ganz oder teilweise zurückzuführen oder vollständig aus dem Prozess zu entfernen.

Das in dem Gemisch eventuell noch enthaltene Alkylencarbonat kann mit Wasser in Gegenwart des gegebenenfalls noch vorhandenen homogenen oder in Gegenwart eines heterogenen Katalysators zu Alkylenglykol umgesetzt werden (z. B. EP 889025). Weiterhin besteht auch die Möglichkeit Alkylencarbonat mit Alkylenglykol zu Dialkylenglykol und Kohlendioxid umzusetzen (z. B. EP 1174406). Bevorzugt werden diese Reaktionen in Kolonnen durchgeführt. Die Reaktion zu Dialkylenglykol findet untergeordnet bereits bei den moderaten Temperaturen, bei denen die Umesterung in der Umesterungskolonne erfolgt, statt und kann durch geeignete Wahl (Erhöhung) der Temperatur und Verweilzeit in den Apparaten, denen der Sumpf der Umesterungskolonne zugeführt wird, verstärkt werden.

Bevorzugt wird die weitere Reaktion des Alkylencarbonats nicht forciert. Nach bevorzugt destillativer Abtrennung der leichtsiedenden Komponenten wird aus dem verbleibenden Produktstrom ein Gemisch im wesentlichen bestehend aus Alkylenglykol und Alkylencarbonat abgetrennt und dieses dem Schritt der oxidativen Carbonylierung (Schritt a)) zugeführt.

Da das erfindungsgemäße Verfahren jedoch Verunreinigungen von Dialkylcarbonat im zurückgeführten Alkylencarbonat zulässt, können an dieser Stelle die Aufreinigungsmaßnahmen weniger aufwändig ausfallen als nach dem Verfahren, die aus dem Stand der Technik bekannt sind.

Bevorzugt wird jedoch das Alkylenglykol weniger als 1000 mol ppm, besonders bevorzugt weniger als 500 mol ppm, insbesondere weniger als 200 mol ppm Dialkylcarbonat enthalten

Das in Schritt (e) abgetrennte und gegebenenfalls aufgereinigte Alkylenglycol wird einer oxidativen Carbonylierung unter Anwesenheit von Kohlenmonoxid unterworfen (Schritt (a)) und zu Alkylencarbonat umgesetzt.

Das erfindungsgemäße Verfahren wird bevorzugt wie folgt durchgeführt: die Reaktionsmischung wird auf die gewünschte Reaktionstemperatur erwärmt und die Reaktion gestartet, indem man ein Kohlenmonoxid/Sauerstoff-Gasgemisch in die Reaktionslösung einleitet. Die Schritte können auch in umgekehrter Reihenfolge durchgeführt werden.

Die Zusammensetzung des Gasgemisches kann in weiten Grenzen beliebig gewählt werden. Statt Sauerstoff kann ein Sauerstoff enthaltendes Gasgemisch, wie beispielsweise auch Luft verwendet werden, oder das Gasgemisch gegebenenfalls mit einem Inertgas wie Stickstoff, Argon oder Kohlendioxid verdünnt werden. Das Verhältnis von Kohlenmonoxyd, Sauerstoff und Inertgas liegt bevorzugt außerhalb des explosiven Bereichs. Im allgemeinem wird ein CO/O₂-Molverhältnis von 100:1 bis 0,01:1, bevorzugt 50:1 bis 0,1:1, besonders bevorzugt 8:1 bis 1:1 verwendet. Bei Verwendung von Inertgasen beträgt das Partialdruckverhältnis der Aktivgase zu Inertgas 98:2 bis 2:98, vorzugsweise 95:5 bis 20:80, besonders bevorzugt 95:5 bis 50:50.

Die Reaktionsgase werden in die im Reaktor befindliche Flüssigkeit eingeleitet. Zur besseren Durchmischung und Dispergierung der Reaktionsgase im Reaktor kann in einer bevorzugten Ausführungsform mechanisch gerührt werden. Als Reaktortyp eignen sich Rührkessel, Rührautoklaven oder Blasenreaktoren oder Blasensäulen, die zweckmäßigerweise beheizt werden können und gewünschtenfalls zusätzlich mit einer Rührvorrichtung versehen sein können.

Das erfindungsgemäße Verfahren kann in einem weiten Druck- und Temperaturbereich durchgeführt werden. Im Allgemeinen kann die Temperatur im Bereich von 0 bis 200°C liegen, vorzugsweise von 60 bis 150°C, besonders bevorzugt von 80 bis 120°C. Die Umsetzung wird zweckmäßigerweise unter erhöhtem Druck, im Allgemeinen bei einem Druck von 1 bis 200 bar, bevorzugt 5 bis 100 bar, besonders bevorzugt 10 bis 30 bar durchgeführt.

Die Reaktion wird in einer bevorzugten Ausführungsform zweckmäßigerweise unter Anwendung des gleichen Diols, Polyols oder Carbonats als Lösungsmittel ausgeführt. In einer alternativen Ausführungsform oder bei Verwendung fester Glykole kann das Glykol mit einem inerten Lösungsmittel verdünnt oder in einem inerten Lösungsmittel gelöst werden. Geeignete inerte Lösungsmittel sind aliphatische, aromatische oder halogenierte Kohlenwasserstoffe, Ether, Ester oder Carbonate. Bevorzugte Lösungsmittel sind Chlorbenzol, Dichlorbenzol, Toluol, Xylol, DMF (Dimethylformamid, Dimethylacetamid, Tetrahydrofuran, NMP (N-Methyl-2-pyrrolidon), DME (Dimethylether), Ethylacetat, Ethylencarbonat oder Propylencarbonat.

Die Umsetzung kann kontinuierlich oder diskontinuierlich durchgeführt werden. Bei der bevorzugten kontinuierlichen Betriebsweise werden die Reaktionsgase in der Regel im Überschuss verwendet und das nicht umgesetzte Gas im Kreis gefahren.

Als Redoxkatalysator wird wenigstens ein Edelmetall, ausgewählt aus Palladium, Rhodium, Iridium und Platin in der elementaren Form oder als ihre ionischen oder nicht-ionischen Verbindungen eingesetzt. Bevorzugt wird das Edelmetall als in der Reaktionsmischung lösliche Verbindung eingesetzt. Besonders eignen sich beispielsweise die Salze oder die Organometallischen Verbindungen von Palladium in der Oxidationsstufe II, Rhodium in der Oxidationsstufe I oder III, Iridium in der Oxidationsstufe I oder III oder Platin in der Oxidationsstufe II. Die Edelmetalle können jedoch auch in einer anderen Oxidationsstufe und als geträgerters oder nicht geträgertes fein disperses Metall eingesetzt werden. Besonders bevorzugt wird als Edelmetall Palladium eingesetzt, wobei es entweder elementar oder als Verbindung vorliegen kann. Werden Salze verwendet, so eignen sich alle Anionen als Gegenion, die unter Reaktionsbedingungen stabil sind und nur schwach an das Metallion koordiniert sind. Beispiele für solche Salze sind die Sulfate, Sulfonate, Chloride, Bromide, Acetate und Trichloroacetate der oben genannten Edelmetalle.

Als redoxaktiver Cokatalysator wird wenigstens eine Verbindung ausgewählt aus Mangan-, Kobalt- oder Kupferverbindungen, in einem Gewichtsverhältnis Katalysatorverbindung zu redoxaktiver Substanz von 1:1 1 bis 1:100, vorzugsweise 1:2 bis 1:30 eingesetzt. Beispiele für Mangan-, Kobalt- oder Kupferverbindungen gemäß dem erfindungsgemäßen Verfahren sind deren Oxide, Sulfate, Halogenide, Acetylacetonate oder Carboxylate.

Zusätzlich können eine Base, Bromidquellen, quaternäre Salze, verschiedene Chinone bzw. Hydrochinone und Trockenmittel eingesetzt werden. Ferner können andere redoxaktive Substanzen, wie Chinone, Alkali- oder Erdalkalliodide, in einem Gewichtsverhältnis Katalysatorverbindung zu redoxaktiver Substanz von 1:1 bis 1:100, vorzugsweise 1:2 bis 1:30, anwesend sein. Beispiele für Chinone sind Benzochinon, Naphthochinon und Anthrachinon, sowie deren Substitutionsprodukte.

Als weitere Hilfsmittel können Oniumsalze, wie Ammonium-, Phosphonium- oder Sulfoniumsalze, Bleiverbindungen, wie Bleialkyle oder -oxide, Polymere, wie Polyvinylpyrrolidon, Alkylhalogenide, wie Dibromethan, in einem Gewichtsverhältnis von Katalysatorverbindung zu Hilfsmittel von 1:1 bis 1:10'000, vorzugsweise 1:10 bis 1:1000, eingesetzt werden. Besonders bevorzugt werden Kaliumbromid oder die Bromide der Ammonium- oder Phosphoniumverbindungen in einem Gewichtsverhältnis von Katalysatorverbindung zu Hilfsmittel von 1:1 bis 1:10'000, vorzugsweise 1:10 bis 1:1000, eingesetzt.

Im Rahmen der Erfindung in Schritt (g) und (h) hergestellte Diarylcarbonate sind bevorzugt solche der allgemeinen Formel (VII) 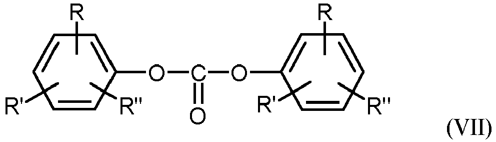 wobei R, R' und R" unabhängig voneinander H, lineares oder verzweigtes, gegebenenfalls substituiertes C₁-C₃₄-Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl, C₆-C₃₄-Aryl oder einen Halogenrest, bevorzugt einen Chlorrest darstellen und R, R' und R" auf beiden Seiten der Formel (VII) gleich oder verschieden sein können. R kann auch -COO-R"' bedeuten, wobei R"' H, gegebenenfalls verzweigtes C₁-C₃₄ Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl oder C₆-C₃₄-Aryl sein kann. Bevorzugt sind R, R' und R" auf beiden Seiten der Formel (VII) gleich. Ganz besonders bevorzugt stehen R, R' und R" für H.

Diarylcarbonate der allgemeinen Formel (VII) sind beispielsweise: Diphenylcarbonat, Methylphenyl-phenyl-carbonate und Di-(methylphenyl)-carbonate, auch als Gemisch, wobei die Stellung der Methylgruppe an den Phenylringen beliebig sein kann, sowie Dimethylphenyl-phenyl-carbonate und Di-(dimethylphenyl)-carbonate, auch als Gemisch, wobei die Stellung der Methylgruppen an den Phenylringen beliebig sein kann, Chlorphenyl-phenyl-carbonate und Di-(chlorphenyl)-carbo-nate, wobei die Stellung der Methylgruppe an den Phenylringen beliebig sein kann, 4-Ethyl-phenyl-phenyl-carbonat, Di-(4-ethylphenyl)-carbonat, 4-n-Propylphenyl-phenyl-carbonat, Di-(4-n-propylphenyl)-carbonat, 4-iso-Propylphenyl-phenyl-carbonat, Di-(4-iso-propylphenyl)-carbonat, 4-n-Butylphenyl-phenyl-carbonat, Di-(4-n-butylphenyl)-carbonat, 4-isoButylphenyl-phenyl-carbo-nat, Di-(4-iso-butylphenyl)-carbonat, 4-tert-Butylphenyl-phenyl-carbonat, Di-(4-tert-butylphenyl)-carbonat, 4-n-Pentylphenyl-phenyl-carbonat, Di-(4-n-pentylphenyl)-carbonat, 4-n-Hexylphenyl-phenyl-carbonat, Di-(4-n-hexylphenyl)-carbonat, 4-iso-Octylphenyl-phenyl-carbonat, Di-(4-iso-octylphenyl)-carbonat,4-n-Nonylphenyl-phenyl-carbonat, Di-(4-n-nonylphenyl)-carbonat, 4-Cyclohexylphenyl-phenyl-carbonat, Di-(4-cyclohexylphenyl)-carbonat, 4-(1-Methyl-1-phenyl-ethyl)-phenyl-phenyl-carbonat, Di-[4-(1-methyl-1-phenylethyl)-phenyl]-carbonat, Biphenyl-4-yl-phenyl-carbonat, Di-(biphenyl-4-yl)-carbonat, (1-Naphthyl)-phenyl-carbonat, (2-Naphthyl)-phenyl-carbonat, Di-(1-naphthyl)-carbonat, Di-(2-naphthyl)-carbonat, 4-(1-Naphthyl)-phenyl-phenyl-car-bonat, 4-(2-Naphthyl)-phenyl-phenyl-carbonat, Di-[4-(1-naphthyl)phenyl]-carbonat, Di-[4-(2-naph-thyl)phenyl]-carbonat, 4-Phenoxyphenyl-phenyl-carbonat, Di-(4-phenoxyphenyl)-carbonat, 3-Pen-tadecylphenyl-phenyl-carbonat, Di-(3-pentadecylphenyl)-carbonat, 4-Tritylphenyl-phenyl-carbo-nat, Di-(4-tritylphenyl)-carbonat, Methylsalicylat-phenyl-carbonat, Di-(methylsalicylat)-carbonat, Ethylsalicylat-phenyl-carbonat, Di-(ethylsalicylat)-carbonat, n-Propylsalicylat-phenyl-carbonat, Di-(n-propylsalicylat)-carbonat, iso-Propylsalicylat-phenyl-carbonat, Di-(iso-propylsalicylat)-carbonat, n-Butylsalicylat-phenyl-carbonat, Di-(n-butylsalicylat)-carbonat, iso-Butylsalicylat-phenyl-carbo-nat, Di-(iso-butylsalicylat)-carbonat, tert-Butylsalicylat-phenyl-carbonat, Di-(tert-butylsalicylat)-carbonat, Di-(phenylsalicylat)-carbonat und Di-(benzylsalicylat)-carbonat.

Bevorzugte Diarylcarbonate sind: Diphenylcarbonat, 4-tert-Butylphenyl-phenyl-carbonat, Di-(4-tert-butylphenyl)-carbonat, Biphenyl-4-yl-phenyl-carbonat, Di-(biphenyl-4-yl)-carbonat, 4-(1-Methyl-1-phenylethyl)-phenyl-phenyl-carbonat und Di-[4-(1-methyl-1-phenylethyl)-phenyl]-carbonat.

Besonders bevorzugt ist Diphenylcarbonat.

Im erfindungsgemäßen Verfahren werden die aromatische(n) Hydroxyverbindung(en) und das oder die Dialkylcarbonat(e) bevorzugt im Molverhältnis von 1 : 0.1 bis 1 : 10, besonders bevorzugt von 1 : 0.2 bis 1 : 5, ganz besonders bevorzugt von 1 : 0.5 bis 1 : 3 in der ersten Reaktionskolonne eingesetzt. Dabei berücksichtigt das angegebene Molverhältnis nicht die Rückführung von aromatischer Hydroxyverbindung oder Dialkylcarbonat in die Reaktionskolonne über einen oder mehrere Kopfkondensator(en) oder einen oder mehrere etwaig vorhandene(n) Sumpfverdampfer.

Im Rahmen der Erfindung geeignete aromatische(n) Hydroxyverbindung(en) (Monophenole) die in Schritt (g) verwendet werden sind bevorzugt solche der allgemeinen Formel (VIII) 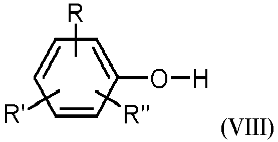 worin R, R' und R" unabhängig voneinander die für die allgemeine Formel (VII) genannte Bedeutung haben können.

Solche aromatischen Monophenole sind beispielsweise: Phenol, o-, m- oder p-Kresol, auch als Gemisch der Kresole, Dimethylphenol, auch als Gemisch, wobei die Stellung der Methylgruppen am Phenolring beliebig sein kann, z.B. 2,4-, 2,6-, oder 3,4-Dimethylphenol, o-, m- oder p-Chlorphenol, o-, m- oder p-Ethylphenol, o-, m- oder p-n-Propylphenol, 4-iso-Propylphenol, 4-n-Butylphenol, 4-iso-Butylphenol, 4-tert-Butylphenol, 4-n-Pentylphenol, 4-n-Hexylphenol, 4-iso-Octylphenol, 4-n-Nonylphenol, o-, m- oder p-Methoxyphenol, 4-Cyclohexylphenol, 4-(1-Methyl-1-phenylethyl)-phenol, Biphenyl-4-ol, 1-Naphthol, 2-1-Naphthol, 4-(1-Naphthyl)phenol, 4-(2-Naphthyl)-phenol, 4-Phenoxyphenol, 3-Pentadecylphenol, 4-Tritylphenol, Methylsalicylsäure, Ethylsalicylsäure, n-Propylsalicylsäuret, iso-Propylsalicylsäure, n-Butylsalicylsäure, iso-Butylsali-cylsäure, tert-Butylsalicylsäure, Phenylsalicylsäure und Benzylsalicylsäure.

Bevorzugte Monophenole sind Phenol, 4-tert-Butylphenol, Biphenyl-4-ol und 4-(1-Methyl-1-phenylethyl)-phenol.

Besonders bevorzugt ist Phenol.

Im Rahmen der Erfindung in Schritt (g) als Zwischenprodukte erhaltene Alkylarylcarbonate sind bevorzugt solche der allgemeinen Formel (IX) 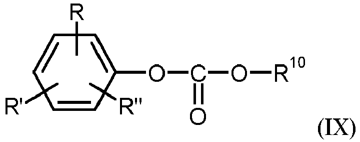 worin R, R' und R" die für die allgemeine Formel (VII) und R¹⁰ die für die allgemeine Formel (II) genannte Bedeutung haben können.

Bevorzugte Alkylarylcarbonate sind Methylphenylcarbonat, Ethylphenylcarbonat, Propylphenylcarbonat, Butylphenylcarbonat, Ethyl-(p-kresyl)-carbonat, Methyl- oder Ethyl-(p-chlorphenyl)-carbonat, Hexylphenylcarbonat, Methyl-(o-kresyl)-carbonat, Methyl-(p-kresyl)-carbonat, Ethyl-(o-kresyl)-carbonat. Besonders bevorzugte Alkylarylcarbonate sind Methylphenylcarbonat, Ethylphenylcarbonat und Butylphenylcarbonat. Ganz besonders bevorzugt sind Methylphenylcarbonat und Ethylphenylcarbonat.

Als Katalysatoren für die Umesterung von Dialkylcarbonaten und/oder Alkylarylcarbonaten mit Monophenolen sind Alkalihydroxide, Lewissäuren aus der Gruppe der Metallhalogenide (DE-OS 2 528 412), Organozinnverbindungen (EP 879 A1, EP 880 A1, DE-OS 3 445 552, EP 338 760 A1), Bleiverbindungen (JP 57/176 932), Lewissäure/-Protonensäure-Katalysatoren (DE-OS 3 445 553) zu nennen.

Das erfindungsgemäße Verfahren zur Herstellung von Diarylcarbonaten in Schritt (g) und (h) wird in mindestens zwei Reaktionskolonnen durchgeführt.

Als erste und zweite Reaktionskolonne oder gegebenenfalls dritte bzw. weitere Kolonne(n) kommen dem Fachmann bekannte Kolonnen in Frage. Dies sind beispielsweise Destillations- bzw. Rektifikationskolonnen, bevorzugt Reaktivdestillations- bzw. Reaktivrektifikationskolonnen.

Die erste Reaktionskolonne enthält wenigstens einen Verstärkungsteil im oberen Teil der Kolonne und wenigstens eine Reaktionszone unterhalb des Verstärkungsteils, welcher mindestens zwei Sektionen aufweist. Jede der zwei Sektionen weist unabhängig voneinander bevorzugt jeweils 0 bis 20, bevorzugt 0,1 bis 20 theoretische Stufen auf. In bevorzugten Ausführungsformen ist wenigstens ein Verstärkungsteil der ersten Reaktionskolonne mit wenigstens einem Zwischenkondensator ausgestattet. Der Zwischenkondensator ist vorzugsweise zwischen den beiden Sektionen des Verstärkungsteils angebracht. In diesem Fall wird der Verstärkungsteil in einen oberen und einen unteren Verstärkungsteil geteilt.

Die erste Reaktionskolonne wird bevorzugt im Gegenstrom betrieben, wobei vorzugsweise in wenigstens einer Reaktionszone dieser Kolonne die aromatische Hydroxyverbindung flüssig vom Kopf zum Sumpf geführt und das Dialkylcarbonat gasförmig diesem flüssigen Strom entgegengeführt wird. Dabei wird die erste Reaktionskolonne vorzugsweise so betrieben, dass man wenigstens einer Reaktionszone, bevorzugt in das obere Drittel der Reaktionszone, einen oder mehrere, die aromatische Hydroxyverbindung und gegebenenfalls gelösten Umesterungskatalysator enthaltende Ströme, bevorzugt mit der an dieser Stelle der Kolonne herrschenden Temperatur, flüssig oder mit nur geringem Gasanteil eindosiert, wobei der Gasanteil bevorzugt weniger als 20 Gew.-% beträgt. Zudem werden einer oder mehrere, das Dialkylcarbonat enthaltende Ströme in die Reaktionszone, bevorzugt im unteren Drittel dieser Reaktionszone, gleitet, wobei die Zudosierung vorzugsweise gasförmig oder überhitzt erfolgt. In bevorzugten Ausführungsformen kann die Überhitzung des Dampfstroms 0 bis 50 °C betragen. Des Weiteren richtet sich die Taupunktstemperatur bevorzugt nach dem in der Reaktionszone an der Zudosierstelle des jeweiligen Dialkylcarbonat enthaltenden Stromes vorliegenden Druck.

Nach Passieren der Reaktionszone(n) wird der während der Reaktion gebildete Alkylalkohol, nach Durchlaufen des oder der Verstärkungsteile, am Kopf der ersten Reaktionskolonne entnommen. Beim während der Reaktion gebildeten Alkylalkohol, auch Reaktionsalkohol genannt, handelt es sich im Rahmen der Erfindung um den bei der Umesterung frei werdenden Alkohol, bevorzugt R¹-OH bzw. R²-OH, wobei R¹ und R² die für die allgemeine Formel (II) genannte Bedeutung haben. Der am Kopf der ersten Reaktionskolonne entnommene Strom enthält im Allgemeinen zusätzlich zum während der Reaktion gebildeten Alkylalkohol noch überschüssiges oder nicht umgesetztes Dialkylcarbonat und leichtsiedende Nebenverbindungen, wie beispielsweise Kohlendioxid oder Dialkylether. Aufgrund des oder der vorhandenen Verstärkungsteil(e) enthält dieser Strom nur geringe Mengen an höher siedenden Komponenten, wie z.B. der aromatischen Hydroxyverbindung. Der Verstärkungsteil dient zur Abtrennung der in der Reaktionszone mitverdampften höher siedenden Komponenten, wie z.B. der aromatischen Hydroxyverbindung oder Alkylarylcarbonat von den leichtsiedenden Reaktionsalkoholen oder Dialkylcarbonaten. Dies hat den Vorteil, dass die Trennung der während der Reaktion gebildeten Alkylalkohole von den Dialkylcarbonaten bei einem niedrigen Temperaturniveau durchgeführt werden kann.

Die erste Reaktionskolonne wird in bevorzugten Ausführungsformen unter Rückflussbedingungen betrieben. Unter Rückflussbedingungen ist eine solche Fahrweise zu verstehen, bei der man den Dampfstrom am oberen Ende des Verstärkungsteils teilweise oder vollständig kondensiert und das dabei anfallende Kondensat teilweise oder vollständig wieder als Rücklauf auf das obere Ende des Verstärkungsteils zurückführt. Das Rücklaufverhältnis beträgt dabei bevorzugt 0,1 bis 20, besonders bevorzugt 0,1 bis 10 und ganz besonders bevorzugt 0,1 bis 3, wobei das Rücklaufverhältnis im Rahmen der Erfindung dem Gewichtsverhältnis von in die Kolonne zurückgeführtem Kondensat zu am Kopf der Kolonne entnommenem Dampf ohne zurückgeführtes Kondensat entspricht.

In bevorzugten Ausführungsformen weist die erste Reaktionskolonne unterhalb einer Reaktionszone wenigstens einen Abtriebsteil auf.

Die erste Reaktionskolonne kann weiterhin bevorzugt mit einem oder mehreren Sumpfverdampfer(n) ausgestattet sein. Bei Ausführung der ersten Reaktionskolonne mit einem Abtriebsteil wird bevorzugt zusätzlich ein Sumpfverdampfer eingesetzt, welcher die aus dem Abtriebsteil ablaufende Flüssigkeit ganz oder teilweise verdampft. Dieser ganz oder teilweise verdampfte Flüssigkeitsstrom wird ganz oder teilweise wieder in die erste Reaktionskolonne zurückgeführt. Im Falle einer Ausführungsform ohne Abtriebsteil wird in einem gegebenenfalls eingesetzten Sumpfverdampfer die aus der Reaktionszone ablaufende Flüssigkeit ganz oder teilweise verdampft und ganz oder teilweise wieder in die erste Reaktionskolonne zurückgeführt. Weiterhin bevorzugt kann die erste Reaktionskolonne im Bereich des Abtriebsteils und/oder der Reaktionszone einen oder mehrere Zwischenerhitzer oder Zwischenverdampfer aufweisen.

In den bevorzugten Ausführungsformen, in denen wenigstens ein Verstärkungsteil der ersten Reaktionskolonne mit wenigstens einem Zwischenkondensator ausgestattet ist, ist der Verstärkungsteil der ersten Reaktionskolonne, der mit wenigstens einem Zwischenkondensator ausgestattet ist, in einen unteren und einen oberen Verstärkungsteil (zwei Sektionen) unterteilt, wovon sich der untere Verstärkungsteil unterhalb des Zwischenkondensators und der obere Verstärkungsteil oberhalb des Zwischenkondensators befinden.

Der oder die Verstärkungsteil(e) mit wenigstens einem Zwischenkondensator können in bevorzugten Ausführungsformen zusammen mit dem oder den Reaktionsteil(en) und gegebenenfalls wenigstens einem Abtriebsteil in der Reaktionskolonne untergebracht werden. Dabei wird das aus der oder den Reaktionszone(n) kommende dampfförmige Gemisch von unten in eine untere Sektion des Verstärkungsteils bzw. gegebenenfalls den unteren Verstärkungsteil geleitet, wobei eine Abreicherung der aromatischen Hydroxyverbindung stattfindet. Das aus dieser unteren Sektion bzw. gegebenenfalls dem unteren Verstärkungsteil kommende dampfförmige Gemisch wird in einen Zwischenkondensator geleitet, wo dieses teilweise auskondensiert und das anfallende Kondensat am oberen Ende der unteren Sektion des Verstärkungsteils bzw. gegebenenfalls dem unteren Verstärkungsteil zugeführt wird.

In einer weiteren bevorzugten Ausführungsform des erfindungsgemäßen Verfahrens wird der Zwischenkondensator nicht in die erste Reaktionskolonne integriert sondern als separater Zwischenkondensator außerhalb der ersten Reaktionskolonne ausgeführt.

In einer weiteren bevorzugten Ausführungsform des erfindungsgemäßen Verfahrens werden Zwischenkondensator und die obere Sektion des Verstärkungsteils nicht in die Reaktionskolonne integriert sondern separat außerhalb der ersten Reaktionskolonne untergebracht.

Unterhalb der Reaktionszone und eines gegebenenfalls vorhandenen Abtriebsteils wird ein Gemisch enthaltend Alkylarylcarbonat, überschüssigem oder nichtumgesetztem Phenol, Diarylcarbonat, Umesterungskatalysatoren, Dialkylcarbonat, Reaktionsalkohol und bei der Reaktion entstehende oder bereits in den Edukten enthaltene schwersiedende Verbindungen erhalten. Bei Verwendung eines Abtriebsteils wird der Gehalt an leichtsiedenden Verbindungen, wie beispielsweise Dialkylcarbonat und Reaktionsalkohol reduziert, wobei in Anwesenheit des Umesterungskatalysators unter Umständen weiteres Alkylarylcarbonat und/oder Diarylcarbonat gebildet werden. Die hierzu erforderliche Energie, wird bevorzugt durch einen oder mehrere Verdampfer zugeführt.

In allen Abschnitten der ersten Reaktionskolonne sowie auch der im Folgenden beschriebenen Kolonnen, d.h. sowohl in Verstärkungs- und/oder gegebenenfalls Abtriebsteil und/oder in der Reaktionszone, können zur Erreichung der betreffenden Trennleistung Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl" Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novaloxringe, Berlsättel, BX-Packungen, Montz Pak, Mellapak, Melladur, Kerapak und CY-Packungen.

Eine Sektion im Rahmen der Erfindung zeichnet sich dadurch aus, dass sich unter- und/oder oberhalb dieser Sektion eine Zufuhr- und/oder Entnahmestelle befindet. Bei der Verwendung von Füllkörperschüttungen und/oder strukturierten Packungen kann eine Sektion in mehrere Abschnitte unterteilt sein, wenn die Sektion mehr als 4, bevorzugt mehr als 10 und besonders bevorzugt mehr als 15 theoretische Stufen aufweist.

Alternativ eignen sich auch Kolonnenböden, wie beispielsweise Sieb-, Glocken-, Ventil-, Tunnelböden. In der oder den Reaktionszone(n) der Reaktionskolonne sind Kolonnenböden mit hohen Verweilzeiten bei gutem Stoffaustausch, beispielsweise Glockenböden, Ventil- oder Tunnelböden mit hohen Überlaufwehren, besonders bevorzugt.

Die theoretische Bodenzahl der Reaktionszone der ersten Reaktionskolonne beträgt vorzugsweise 3 bis 50, besonders bevorzugt 10 bis 50 und ganz besonders bevorzugt 10 bis 40. Der Flüssigkeits-Hold-up beträgt vorzugsweise 1 bis 80%, besonders bevorzugt 5 bis 70% und ganz besonders bevorzugt 7 bis 60% des Kolonneninnenvolumens der Reaktionszone. Die genauere Auslegung der Reaktionszone(n), des gegebenenfalls zu verwendendes Abtriebsteils und des oder der Verstärkerteil(e) kann vom Fachmann vorgenommen werden.

Bei der ersten Reaktionskolonne richtet sich der Kolonnendurchmesser im Bereich der Reaktionszone allerdings nur mit Einschränkungen nach dem Gasdurchsatz. Er wird ferner beeinflusst durch den zu realisierenden Hold-up.

Bei Verwendung von Verweilzeitböden sollte der Flüssigkeitsstand auf den Böden vorzugsweise 50 bis 1000, besonders bevorzugt 100 bis 500 und ganz besonders bevorzugt 100 bis 250 mm betragen um den Druckverlust der Kolonne auf ein sinnvolles Maß zu begrenzen. Der Druckverlust der Kolonne sollte vorzugsweise weniger als 50, besonders bevorzugt weniger als 30 und ganz besonders bevorzugt weniger als 25 % des Kopfdruckes betragen.

Unter diesen Randbedingungen liegt der F-Faktor in der Kolonne vorzugsweise zwischen 0,05 und 2,5, bevorzugt 0,05 bis 1,5 und besonders bevorzugt zwischen 0,08 und 1 Pa^{0,5}. Der Bodenabstand kann vorzugsweise 250 bis 1500 mm, besonders bevorzugt 300 bis 1000 und ganz besonders bevorzugt 500 bis 1000 mm betragen. Der F-Faktor ist ein Maß für die gasseitige hydrodynamische Belastung der Kolonne und berechnet sich wie folgt:
F-Faktor = Gasdichte^{½} • Gasgeschwindigkeit

Ein geeignetes Kolonnendesign der restlichen im Verfahren eingesetzten Destillations- und/oder Reaktionskolonnen, welches sowohl die Festlegung der Kolonnenhöhe, des Kolonnendurchmessers, die Auswahl der Kolonneneinbauten als auch die Dimensionierung der Zulauf- und Entnahmeleitungen umfasst, ist dem Fachmann bekannt und kann der einschlägigen Literatur (z.B. Distillation Design, Henry Z. Kister, Mc Gra Hill; Distillation Operation, Henry Z. Kister, Mc Gra Hill; Perry's Chemical Engineering Handbook; Perry & Green) entnommen werden.

Die Temperatur der Reaktionszone(n) liegt bevorzugt im Bereich von 100 bis 300°C, besonders bevorzugt von 120 bis 250°C, ganz besonders bevorzugt von 150 bis 240°C. In bevorzugten Ausführungsformen wird in der Reaktionszone eine optimale Reaktionstemperatur einerseits durch die Wahl der Betriebsbedingungen und andererseits durch zusätzliche Wärmzufuhr im Bereich eines oder mehrerer Reaktionsböden eingestellt. Die Wärmezufuhr auf den Reaktionsböden kann dabei entweder durch Wärmetauscher oder über Reaktionsböden mit Möglichkeit zum Wärmeeintrag erfolgen. Es ist von Vorteil, die erfindungsgemäße Umesterung nicht nur bei Normaldruck, sondern auch bei erhöhtem oder erniedrigtem Druck durchzuführen. Der Druck der Reaktionszone liegt daher bevorzugt im Bereich von 0.5 bis 20 bar (absolut), besonders bevorzugt von 0.8 bis 15 bar (absolut), ganz besonders bevorzugt von 0.9 bis 10 bar (absolut).

Für die in der ersten Reaktionskolonne auftretenden Reaktionsschritte können aus der Literatur bekannte Umesterungskatalysatoren eingesetzt werden. Dies sind aus der Literatur bekannte Umesterungskatalysatoren für die Dialkylcarbonat-Phenol-Umesterung, wie AlX₃, TiX₃, UX₄, TiX₄, VOX₃, VX₅, ZnX₂, FeX₃, PbX₂ und SnX₄, worin X für Halogen-, Acetoxy-, Alkoxy- oder Aryloxyreste steht (DE-OS 2 58 412). Besonders bevorzugte erfindungsgemäß einsetzbare Katalysatoren sind Metallverbindungen wie AlX₃, TiX₄, PbX₂ und SnX₄, wie beispielsweise Titantetrachlorid, Titantetramethoxid Titantetraphenoxid, Titantetraethoxid, Titantetraisopropylat, Titantetradodecylat, Zinntetraisooctylat und Aluminiumtriisopropylat. Ganz besonders bevorzugt sind Metallverbindungen TiX₄. Die genannten Metallverbindungen werden bevorzugt in Mengen von 0,001 bis 5 Gew.-%, bevorzugt von 0,005 bis 5 Gew.-% und besonders bevorzugt von 0,01 bis 5 Gew.-%, bezogen auf das Gewicht des umzusetzenden Reaktionsgemischs, eingesetzt.

Halogen bedeutet im Rahmen der Erfindung Fluor, Chlor oder Brom, bevorzugt Fluor oder Chlor, besonders bevorzugt Chlor.

Weitere erfindungsgemäß einsetzbare Katalysatoren sind zinnorganische Verbindungen der allgemeinen Formel (R¹¹)_{4-X}-Sn(Y)ₓ, in der Y für einen Rest OCOR¹², OH oder OR steht, wobei R¹² C₁-C₁₂-Alkyl, C₆-C₁₂-Aryl oder C₇-C₁₃-Alkylaryl bedeutet, R¹¹ unabhängig von R¹² die Bedeutung von R¹² hat und x eine ganze Zahl von 1 bis 3 bedeutet, Dialkylzinnverbindungen mit 1 bis 12 C-Atomen im Alkylrest oder Bis-(trialkylzinn)verbindungen, beispielsweise Trimethylzinnacetat, Triethylzinnbenzoat, Tributylzinnacetat, Triphenylzinnacetat, Dibutylzinndiacetat, Dibutylzinndilaurat, Dioctylzinndilaurat, Dibutylzinnadipinat, Dibutyldimethoxyzinn, Dimethylzinnglykolat, Dibutyldiethoxyzinn, Triethylzinnhydroxid, Hexaethylstannoxan, Hexabutylstannoxan, Dibutylzinnoxid, Dioctylzinnoxid, Butylzinntriisooctylat, Octylzinntriisooctylat, Butylstannonsäure und Octylstannonsäure in Mengen von 0,001 bis 20 Gew: % (vgl. EP 879, EP 880, EP 39 452, DE-OS 34 45 555, JP 79/63023), polymere Zinnverbindungen der Formel -[-RR¹¹Sn-O-]-, in welcher R und R¹¹ unabhängig voneinander die vorangehend für R¹² genannte Bedeutung haben, beispielsweise Poly[oxy(dibutylstannylen)] Poly[oxy(dioctylstannylen)], Poly[oxy(butylphenyl-stannylen)] und Poly[oxy(diphenylstannylen)] (DE-OS 34 45 552), polymere Hydroxystannoxane der Formel -[-RSn(OH)-O-]-, beispielsweise Poly(ethylhydroxystannoxan), Poly(butylhydroxy-stannoxan), Poly-(octylhydroxystannoxan), Poly(undecylhydroxystannoxan) und Poly(dodecyl-hydroxystannoxane) in Mengen von 0,001 bis 20 Gew.-%, bevorzugt von 0,005 bis 5 Gew.-%, bezogen auf Dialkylcarbonat (DE-OS 40 06 520). Weitere erfindungsgemäß einsetzbare Zinnverbindungen sind Sn(II)oxide der allgemeinen Formel

X-R₂Sn-O-R₂Sn-Y,

worin X und Y unabhängig voneinander OH, SCN, OR¹³, OCOR¹³ oder Halogen und R Alkyl, Aryl bedeuten soll, worin R¹³ die vorangehend für R¹² genannte Bedeutung hat (EP 0 338 760).

Als weitere erfindungsgemäß einsetzbare Katalysatoren kommen Bleiverbindungen, gegebenenfalls zusammen mit Triorganophosphanen, einer Chelatverbindung oder einem Alkalimetallhalogenid, beispielsweise Pb(OH)₂-2PbCO₃, Pb(OCO-CH₃)₂, Pb(OCO-CH₃)₂ ·2LiCl, Pb(OCO-CH₃)₂ • 2PPh3 in Mengen von 0,001 bis 1, bevorzugt von 0,005 bis 0,25 Mol pro Mol Dialkylcarbonat (JP 57/176932, JP 01/093580), sowie andere Blei(II)- und Blei(IV)-verbindungen, wie PbO, PbO₂, Mennige, Plumbite, und Plumbate (JP 01/093560), Eisen(III)acetat (JP 61/1 72 852), weiterhin Kupfersalze und/oder Metallkomplexe, beispielsweise von Alkali, Zink, Titan und Eisen (JP 89/005588) in Frage.

Weiterhin sind in den erfindungsgemäßen Verfahren heterogene Katalysatorsysteme einsetzbar. Solche sind beispielsweise Mischoxide aus Silizium und Titan, die durch gemeinsame Hydrolyse von Silizium und Titanhalogeniden erhältlich sind (JP 54/125617) oder Titandioxide mit hoher BET-Oberfläche >20 m²/g (DE-OS 40 36 594)).

Bevorzugte Katalysatoren für das erfindungsgemäße Verfahren sind die vorangehend genannten Metallverbindungen AlX₃, TiX₃, UX₄, TiX₄, VOX₃, VX₅, ZnX₂, FeX₃, PbX₂ und SnX₄. Besonders bevorzugt sind AlX₃, TiX₄, PbX₂ und SnX₄, wovon beispielhaft Titantetrachlorid, Titantetramethoxid Titantetraphenoxid, Titantetraethoxid, Titantetraisopropylat, Titantetradodecylat, Zinntetraisooctylat und Aluminiumtriisopropylat genannt seien. Ganz besonders bevorzugt sind Metallverbindungen TiX₄. Insbesondere bevorzugt sind Titantetramethoxid, Titantetraphenoxid und Titantetraethoxid.

Der Katalysator wird bevorzugt zusammen mit dem Strom enthaltend die aromatische(n) Hydroxyverbindung(en) in gelöster oder suspendierter Form in die erste Reaktionskolonne eingebracht. Alternativ kann der Katalysator auch separat beispielsweise in einem dem Reaktionsalkohol entsprechenden Alkohol oder einem geeigneten inerten Lösungsmittel zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Die für die Reaktion in der ersten Reaktionskolonne erforderliche Energie kann einerseits über innen oder außen liegende Vorrichtungen, wie z.B. Wärmetauscher, Verdampfer und/oder beheizbare Kolonnenböden, erzeugt und/oder andererseits sowohl mit dem flüssigen Strom enthaltend die aromatische(n) Hydroxyverbindung(en) als auch mit dem gasförmig eindosierten, Dialkylcarbonat enthaltenden Strom eingebracht werden. Insbesondere im Bereich der Reaktionszone(n) kann eine Zufuhr von Wärme auf diese Weise erfolgen. Vorzugsweise wird diese Wärme im Bereich der Reaktionszone(n) ganz oder teilweise mittels Verdampfer oder beheizbarer Kolonnenböden zugeführt. Besonders vorteilhaft ist es, die für die Reaktion in der ersten Reaktionskolonne erforderliche Energie wenigstens teilweise sowohl mit dem flüssigen Strom enthaltend die aromatische(n) Hydroxyverbindung(en) als auch mit dem gasförmig eindosierten, Dialkylcarbonat enthaltenden Strom in die ersten Reaktionskolonne und zusätzlich durch innen- und/oder außen liegende Wärmetauscher einzubringen.

Im erfindungsgemäßen Verfahren wird das Sumpfprodukt der ersten Reaktionskolonne einer zweiten Reaktionskolonne zugeführt.

Die zweite Reaktionskolonne enthält wenigstens einen Verstärkungsteil im oberen Teil der Kolonne und wenigstens eine Reaktionszone unterhalb des Verstärkungsteils. Der Verstärkungsteil weist bevorzugt 1 bis 50, besonders bevorzugt 1 bis 25 theoretische Stufen auf.

In der zweiten Reaktionskolonne wird das Sumpfprodukt der ersten Reaktionskolonne, welches bereits gebildetes Alkylarylcarbonat und Diarylcarbonat enthält, flüssig oder als Dampf-Flüssigkeits-Gemisch bevorzugt der Reaktionszone, besonders bevorzugt dem oberen Teil der Reaktionszone, ganz besonders bevorzugt im oberen Drittel der Reaktionszone zugeführt. Dabei wird die zweite Reaktionskolonne vorzugsweise so betrieben, dass man das Alkylarylcarbonat teilweise oder vollständig, beispielsweise durch weitere Umesterung oder Disproportionierung, bevorzugt durch Disproportionierung, zum Diarylcarbonat umsetzt. Zusätzlich zum Sumpfprodukt der ersten Reaktionskolonne können einer oder mehrere, Alkylarylcarbonat enthaltende Ströme flüssig oder als Dampf-Flüssigkeitsgemisch im Bereich der Reaktionszone eindosiert werden. Solche zusätzlichen Alkylarylcarbonat enthaltenden Ströme können beispielsweise aus der weiteren Aufarbeitung stammen und so in das Verfahren zurückgeführt werden.

Am Kopf der zweiten Reaktionskolonne werden nicht umgesetzte aromatische Hydroxyverbindung, Dialkylcarbonat, Reaktionsalkohol, mittelsiedende Nebenverbindungen - wie beispielsweise Alkylarylether - und in geringem Maße leichtsiedende Nebenverbindungen abgetrennt. Im Rahmen der Erfindung sind unter mittelsiedenden Nebenverbindungen solche mit einem Siedepunkt unterhalb dem des Alkylarylcarbonats und oberhalb dem des Dialkylcarbonats zu verstehen. Solche mittelsiedenden Nebenverbindungen sind z.B. Alkylarylether, wie beispielsweise Anisol oder Phenetol. Die in der zweiten Reaktionskolonne abgetrennten mittelsiedenden Nebenverbindungen können in der ersten und/oder zweiten Reaktionskolonne bei der Reaktion entstehen oder bereits durch die Edukte in das Verfahren eingeschleust worden sein.

Der Verstärkungsteil der zweiten Reaktionskolonne dient zur Abtrennung der in der Reaktionszone mitverdampften höher siedenden Komponenten, wie z.B. Alkylarylcarbonat.

Die zweite Reaktionskolonne wird in bevorzugten Ausführungsformen ebenfalls unter solchen für die erste Reaktionskolonne beschriebenen Rückflussbedingungen betrieben.

Die zweite Reaktionskolonne kann unterhalb einer Reaktionszone wenigstens einen Abtriebsteil aufweisen. In bevorzugten Ausführungsformen kann die Reaktionszone der zweiten Reaktionskolonne jedoch gleichzeitig als Abtriebsteil fungieren. Dabei wird das bei der Disproportionierung freigesetzte Dialkylcarbonat, durch Umesterung freigesetzter Reaktionsalkohol und nicht umgesetzte aromatische Hydroxyverbindung abgetrennt und gleichzeitig Diarylcarbonat und das im Wesentlichen durch Disproportionierung abreagierende Alkylarylcarbonat aufkonzentriert.

Die zweite Reaktionskolonne kann weiterhin bevorzugt mit einem oder mehreren Sumpfverdampfer(n) ausgestattet sein.

Weiterhin bevorzugt kann die zweite Reaktionskolonne im Bereich des Abtriebsteil und/oder der Reaktionszone einen oder mehrere Zwischenerhitzer oder Zwischenverdampfer aufweisen.

Prinzipiell kann der Verstärkungsteil der zweiten Reaktionskolonne ebenfalls mit einem oder mehreren Zwischenkondensatoren ausgestattet werden. Hierdurch wird der Verstärkungsteil, in einen unteren und einen oberen Verstärkungsteil (zwei Sektionen) unterteilt, wovon sich der untere Verstärkungsteil unterhalb des Zwischenkondensators und der obere Verstärkungsteil oberhalb des Zwischenkondensators befinden. In einer bevorzugten Ausführungsform weist wird die zweite Reaktionskolonne keinen Zwischenkondensator auf.

Die zweite Reaktionskolonne ist mit einem oder mehreren Kondensatoren ausgestattet. Bevorzugt handelt es sich dabei um einen oder mehrere Kondensatoren am Kopf der zweiten Reaktionskolonne (Kopfkondensator(en)). Besonders bevorzugt wird eine Kaskade von Kopfkondensatoren verwendet.

Im Laufe der Kondensation in dem oder den Kondensator(en) am Kopf der zweiten Reaktionskolonne verarmen die Dämpfe an höher siedenden Komponenten, wie z.B. aromatischer Hydroxyverbindung. Um die anfallende Kondensationswärme im Sinne einer Wärmeintegration besonders effizient nutzen zu können, erfolgt die Kondensation daher bevorzugt mehrstufig, besonders bevorzugt mindestens zweistufig, in bevorzugten Ausführungsformen zwei- oder dreistufig.

Bei der besonders bevorzugten Ausführungsform der zwei- oder dreistufigen Kondensation wird die Kondensationswärme der ersten oder der ersten und zweiten Kondensationsstufe direkt oder indirekt zum Erwärmen eines Stoffstromes oder einer Kolonne innerhalb des Verfahrens verwendet, während die anfallende Kondensationswärme der zweiten oder dritten Kondensationsstufe durch Kühlwasser oder Luftkühlung abgeführt wird.

Die Kondensation am Kopf der zweiten Reaktionskolonne kann in weiteren bevorzugten Ausführungsformen zudem so durchgeführt werden, dass ein Teil der am Kopf der zweiten Reaktionskolonne entnommenen Dämpfe nicht kondensiert wird, um mittelsiedende Nebenverbindungen selektiv ausschleusen zu können.

Unterhalb der Reaktionszone und eines gegebenenfalls vorhandenen Abtriebsteils wird ein Gemisch enthaltend Alkylarylcarbonat, überschüssiger oder nicht umgesetzter aromatischer Hydroxyverbindung, Diarylcarbonat, Umesterungskatalysator(en), Dialkylcarbonat, Reaktionsalkohol und bei der Reaktion entstehende oder bereits in den Edukten enthaltene mittel-oder schwersiedende Nebenverbindungen erhalten. Im Rahmen der Erfindung sind unter schwersiedenden Nebenverbindungen solche mit einem Siedepunkt oberhalb dem des Alkylarylcarbonats zu verstehen. Solche schwersiedenden Nebenverbindungen lassen sich noch in solche, deren Siedepunkt zwischen dem des Alkylarylcarbonats und dem des Diarylcarbonats liegt (Schwersieder), und solche, deren Siedepunkt oberhalb des Siedepunktes des Diarylcarbonats liegt (Hochsieder), unterteilen.

In allen Abschnitten der zweiten Reaktionskolonne, d.h. sowohl in Verstärkungs- und gegebenenfalls Abtriebsteil als auch in der Reaktionszone können die bereits vorangehend für die erste Reaktionskolonne genannten Füllkörper oder geordnete Packungen zum Einsatz kommen.

Die genauere Auslegung der Reaktionszone(n), des gegebenenfalls zu verwendendes Abtriebsteils und des oder der Verstärkerteil(e) kann vom Fachmann vorgenommen werden.

Die Temperatur der Reaktionszone(n) liegt bevorzugt im Bereich von 100 bis 300°C, besonders bevorzugt von 120 bis 250°C, ganz besonders bevorzugt von 180 bis 250°C.

In besonderen Ausführungsformen wird in der Reaktionszone eine optimale Reaktionstemperatur einerseits durch die Wahl der Betriebsbedingungen und andererseits durch zusätzliche Wärmzufuhr im Bereich eines oder mehrerer Reaktionsböden eingestellt. Die Wärmezufuhr auf den Reaktionsböden kann dabei entweder durch Wärmetauscher oder über Reaktionsböden mit Möglichkeit zum Wärmeeintrag erfolgen. Es ist von Vorteil, die erfindungsgemäße Umesterung nicht nur bei Normaldruck, sondern auch bei erhöhtem oder erniedrigtem Druck, bevorzugt bei erniedrigtem Druck, durchzuführen. Der Druck der zweiten Reaktionskolonne liegt daher bevorzugt im Bereich von 0,05 bis 20 bar (absolut), besonders bevorzugt von 0,1 bis 10 bar (absolut), ganz besonders bevorzugt von 0,1 bis 2 bar (absolut).

Für die in der zweiten Reaktionskolonne auftretenden Reaktionsschritte können die vorangehend bereits für die Umesterung in der ersten Reaktionskolonne genannten Umesterungskatalysatoren eingesetzt werden. In einer bevorzugten Ausführungsform werden in der ersten und zweiten Reaktionskolonne identische Katalysatoren verwendet.

Der Katalysator wird bevorzugt zusammen mit dem Sumpfprodukt der ersten Reaktionskolonne in gelöster oder suspendierter Form in die zweite Reaktionskolonne eingebracht. Alternativ kann der Katalysator auch separat beispielsweise in einem dem Reaktionsalkohol entsprechenden Alkohol oder einem geeigneten inerten Lösungsmittel zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Im erfindungsgemäßen Verfahren fallen bei der Umesterung und/oder Disproportionierung in der ersten Reaktionskolonne und/oder der oder den weiteren Reaktionskolonne(n) Ströme enthaltend während der Reaktion gebildeten Alkylalkohol (Reaktionsalkohol) sowie nicht umgesetztes oder während der Reaktion gebildetes Dialkylcarbonat an und werden vorzugsweise in einem oder mehreren Strömen im Gemisch entnommen. Dieses in den Reaktionskolonnen nicht umgesetzte oder während der Reaktion gebildete Dialkylcarbonat wird erfindungsgemäß ganz oder teilweise in wenigstens einem weiteren Verfahrensschritt enthaltend wenigstens eine Destillationskolonne vom während der Reaktion gebildeten Alkylalkohol (Reaktionsalkohol) getrennt. Bevorzugt wird wenigstens ein Strom enthaltend nicht umgesetztes oder während der Reaktion gebildetes Dialkylcarbonat und während der Reaktion gebildeten Alkylalkohol am Kopf der ersten Reaktionskolonne entnommen und zur Auftrennung wenigstens einem weiteren Verfahrensschritt enthaltend wenigstens eine Destillationskolonne zugeführt.

Bevorzugt wird das am Kopf der ersten Reaktionskolonne entnommene Dampfgemisch enthaltend Dialkylcarbonat und während der Reaktion gebildetem Alkylalkohol nach Kondensation am Kopf der ersten Reaktionskolonne ganz oder teilweise wenigstens einem weiteren Verfahrensschritt enthaltend wenigstens eine Destillationskolonne zur Trennung von Dialkylcarbonat und Alkylalkohol - im Folgenden als Trenn-Destillationskolonne(n) bezeichnet - zugeführt. Besonders bevorzugt wird das dabei abgetrennte Dialkylcarbonat gegebenenfalls nach weiterer Aufreinigung wieder der ersten Reaktionskolonne zugeführt.

Die Trennung des Dialkylcarbonats und des Reaktionsalkohols erfolgt bevorzugt destillativ in einer oder mehreren Trenn-Destillationskolonnen oder in einer Kombination aus Destillation und Membrantrennung - im Folgenden als Hybridprozess - bezeichnet.

Bilden Reaktionsalkohol und Dialkylcarbonat ein Azeotrop (z.B. Methanol und Dimethylcarbonat) so wird bevorzugt ein wenigstens zweistufiges Verfahren wie beispielsweise ein Zweidruckverfahren, eine Extraktivdestillation, eine Heteroazeotropdestillation mit einem niedrigsiedenden Schleppmittel oder ein Hybridverfahren verwendet. Besonders bevorzugt wird das Zweidruckverfahren oder ein Hybridverfahren angewendet. Ganz besonders bevorzugt wird das Zweidruckverfahren angewendet. Solche Verfahren sind dem Fachmann grundsätzlich bekannt.

Bilden Reaktionsalkohol und Dialkylcarbonat kein Azeotrop (z.B. Ethanol und Diethylcarbonat), so erfolgt die Trennung bevorzugt in einer einzelnen Trenn-Destillationskolonne.

Bilden Reaktionsalkohol und Dialkylcarbonat ein Azeptrop, so weist das Destillat einer ersten Trenn-Destillationskolonne des Verfahrensschritts zur Trennung von Dialkylcarbonat und Alkylalkohol (Reaktionsalkohol) vorzugsweise nahezu azeotrope Zusammensetzung auf. In diesem Fall wird dieses bevorzugt in einem Zweidruckverfahren wenigstens einer weiteren Trenn-Destillationskolonne zugeführt die bei einem Betriebsdruck arbeitet, der unter dem der ersten Trenn-Destillationskolonne liegt. Durch den unterschiedlichen Betriebsdruck verschiebt sich die Lage des Azeotrops hin zu geringeren Anteilen an Reaktionsalkohol. Man erhält als Sumpfprodukt dieser zweiten oder weiteren Trenn-Destillationskolonne(n) Reaktionsalkohol in einer Reinheit von 90 bis 100 Gew.-%, bezogen auf das Gesamtgewicht des isolierten Sumpfproduktes, und als Destillat eine nahezu azeotropes Gemisch. Die bei geringerem Betriebsdruck arbeitende zweite oder weitere(n) Trenn-Destillationskolonne(n) wird in ganz besonders bevorzugten Ausführungsformen vorzugsweise mit der Kondensationswärme des oder der Kopfkondensator(en) der ersten Trenn-Destillationskolonne betrieben.

Beim Zweidruckverfahren macht man sich die Druckabhängigkeit der azeotropen Zusammensetzung eines Zweistoffgemisches zunutze. Bei einem Gemisch aus Reaktionsalkohol (Alkylalkohol) und Dialkylcarbonat, wie beispielsweise Methanol und Dimethylcarbonat, verschiebt sich die azeotrope Zusammensetzung mit zunehmendem Druck zu höheren Reaktionsalkoholgehalten. Führt man ein Gemisch dieser beiden Komponenten einer ersten Trenn-Destillationskolonne (Dialkylcarbonatkolonne) zu, wobei der Reaktionsalkoholgehalt unterhalb der für den Betriebsdruck dieser Kolonne entsprechende azeotropen Zusammensetzung liegt, so erhält man als Destillat ein Gemisch mit nahezu azeotroper Zusammensetzung und als Sumpfprodukt nahezu reines Dialkylcarbonat. Das so erhaltende azeotrope Gemisch wird einer weiteren Trenn-Destillationskolonne (Alkylalkoholkolonne) zugeführt. Diese arbeitet bei einem im Vergleich zur Dialkylcarbonatkolonne geringeren Betriebsdruck. Dadurch verschiebt sich die Lage des Azeotrops hin zu geringeren Reaktionsalkoholgehalten. Hierdurch ist es möglich, das in der Dialkylcarbonatkolonne erhaltene azeotrope Gemisch in ein Destillat mit nahezu azeotroper Zusammensetzung und nahezu reinen Reaktionsalkohol zu trennen. Das Destillat der Alkylalkoholkolonne wird wieder der Dialkylcarbonatkolonne an geeigneter Stelle zugeführt.

Mit dem erfindungsgemäßen Verfahren können vorzugsweise Diarylcarbonate mit einer Reinheit von, d.h. einem Gehalt von reinem Diarylcarbonat von, 99 bis 100 Gew.-%, besonders bevorzugt 99,5 bis 100 Gew.-% und ganz besonders bevorzugt 99,9 bis 100 Gew.-%, bezogen auf das Gesamtgewicht des aufgereinigten Diarylcarbonats erhalten werden.

Das im Seitenstrom der ersten Diarylcarbonat-Destillationskolonne entnommene Diarylcarbonat kann flüssig oder dampfförmig entnommen werden. Bevorzugt wird das im Seitenstrom der ersten Diarylcarbonat-Destillationkolonne entnommene Diarylcarbonat dampfförmig entnommen. In bevorzugten Ausführungsformen kann jedoch die flüssige Entnahme des Diarylcarbonats im Seitenstrom insbesondere aufgrund konstruktiver Gegebenheiten bevorzugt sein.

Trennwandkolonnen sind dem Fachmann bekannt und beispielsweise in DE-A 33 02 525 oder DE-A 199 14 966 beschrieben.

Im Verfahrensschritt (b) wird ein katalysatorhaltiger Strom erhalten, welcher ganz oder teilweise gegebenenfalls nach weiterer Aufreinigung wieder in das Verfahren, bevorzugt in Verfahrensschritt (a) zurückführt wird. Dadurch können sowohl Verluste teurer Katalysatoren als auch Verluste an gewünschten Diarylcarbonat vermieden und damit dass erfindungsgemäße Verfahren zusätzlich wirtschaftlicher gestaltet werden.
**Fig. 1** zeigt ein integriertes Verfahren zur Herstellung von Polycarbonat und Rückführung des anfallenden Ethylenglykols und des Phenols
**Fig. 2** zeigt eine Ausführungsform des Verfahrens zur Herstellung von Dialkylcarbonat aus Alkylencarbonat.
**Fig. 3** beschreibt eine Ausführungsform des Verfahrens zur Herstellung von Diarylcarbonat aus Dialkylcarbonat.
**Fig. 4** beschreibt eine Ausführungsform der Destillation von Diarylcarbonat

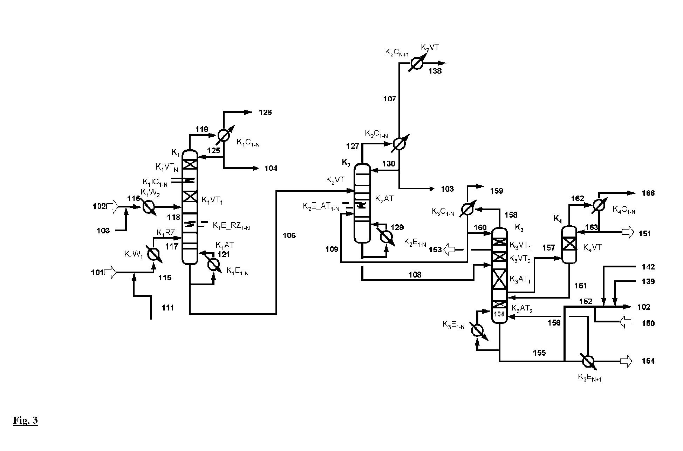

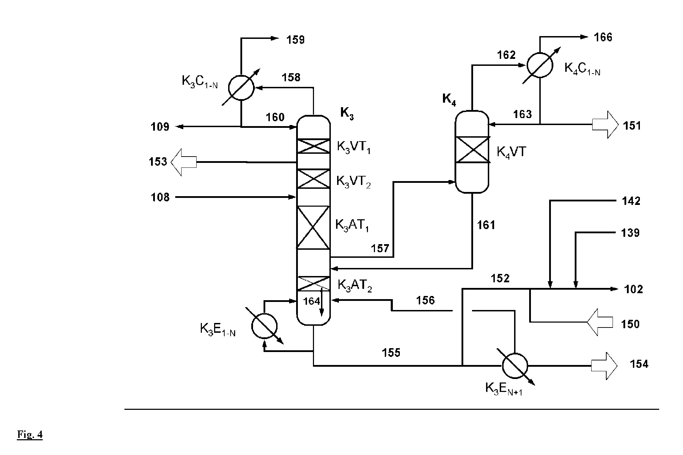

In Fig. 3 und Fig. 4 haben die Bezugszeichen folgende Bedeutungen:
- K₁: Alkylarylcarbonat-Reaktionskolonne (erste Reaktionskolonne)
- K₁C_{1-N}: Kopfkondensator(en) 1 bis N der K₁
- K₁E_{1-N}: Verdampfer 1 bis N der K₁
- K₁IC₁-_{N}: Zwischenkondensator(en) 1 bis N der K₁
- K₁VT₁: unterer Verstärkungsteil der K₁
- K₁VT_{N}: oberer Verstärkungsteil der K₁
- K₁W₁: Vorwärmer/Verdampfer/Überhitzer der K₁ für Dialkylcarbonat enthaltenden Strom
- K₁W₂: Vorwärmer/Verdampfer der K₁ für Eduktstrom mit aromatischer Hydroxyverbindung
- K₁RZ: Reaktionszone der K₁
- K₁AT: Abtriebsteil der K₁
- K₁E_RZ_{1-N}: Zwischenverdampfer 1 bis N im Bereich der Reaktionszone der K₁

- K₂: Diarylcarbonat-Reaktionskolonne (zweite Reaktionskolonne)
- K₂C_{1-N}: Kopfkondensator(en) 1 bis N der K₂
- K₂C_{N+1}: Kondensator für Restbrüdenstrom aus K₂C_{1-N} enthaltend mittelsiedende Nebenverbindungen
- K₂E_{1-N}: Verdampfer 1 bis N der K₂
- K₂VT: Verstärkungsteil der K₂
- K₂AT: Abtriebsteil und Reaktionszone der K₂
- K₂E_AT_{1-N}: Zwischenverdampfer 1 - N im Abtriebsteil der K₂

- K₃: Diarylcarbonat-Feindestillationskolonne (erste Diarylcarbonat-Destillationskolonne)
- K₃C_{1-N}: gegebenenfalls mehrstufige(r) Kopfkondensator(en) 1 bis N der K₃
- K₃E_{1-N}: gegebenenfalls mehrstufige(r) Verdampfer 1 bis N der K₃
- K₃E_{N+1}: Zusätzlicher Verdampfer zur Aufkonzentrierung des nicht in das Verfahren zurückgeführten Sumpfproduktstromes aus dem Sumpf der Kolonne K₃ und/oder der/des Kolonnenverdampfer/s K₃E_{1-N}
- K₃SC_{1-N}: gegebenenfalls mehrstufige(r) Kondensator(en) 1 bis N für dampfförmigen Seitenstrom der K₃
- K₃VT₁: oberer Verstärkungsteil der K₃
- K₃VT₂: unterer Verstärkungsteil der K₃
- K₃AT₁: oberer Abtriebsteil der K₃
- K₃AT₂: unterer Abtriebsteil der K₃
- K₃T: Trennwand der K₃
- K₃TLO: Verstärkungsteil auf der Zulaufseite der Trennwand der K₃
- K₃TLU: Abtriebsteil auf der Zulaufseite der Trennwand der K₃
- K₃TRU: Verstärkungsteil auf der Entnahmeseite der Trennwand der K₃
- K₃TRO: Abtriebsteil auf der Entnahmeseite der Trennwand der K₃
- K₄: Diarylcarbonat-Seitenstromkolonne (zweite Diarylcarbonat-Destillationskolonne)
- K₄C_{1-N}: gegebenenfalls mehrstufige(r) Kopfkondensator(en) 1 bis N der K₄
- K₄VT: Verstärkungsteil der K₄
- K₄AT: Abtriebsteil der K₄
- K₄E_{1-N}: gegebenenfalls mehrstufige(r) Verdampfer 1 bis N der K₄

Weiterhin sind in den Fig. 3 und 4 die folgenden Stoffströme benannt
- 101: Eduktzustrom enthaltend Dialkylcarbonat
- 102: Eduktzustrom enthaltend aromatische Hydroxyverbindung
- 103: Destillat der K₂
- 104: Destillat der K₁
- 105: Strom enthaltend Dialkylcarbonat und Reaktionsalkohol
- 106: Sumpfprodukt der K₁
- 107: Zwischensiederpurge
- 108: Sumpfprodukt der K₂
- 109: Strom enthaltend Alkylarylcarbonat und aromatische Hydroxyverbindung
- 111: Dialkylcarbonat enthaltender Strom
- 115: Dialkylcarbonat enthaltender Strom zur K₁
- 116: Aromatische Hydroxyverbindung enthaltender Strom zur K₁
- 117: Dialkylcarbonat enthaltender Strom nach Verdampfung
- 118: Strom mit aromatischer Hydroxyverbindung nach Erhitzung
- 119: Dampfstrom am Kopf der K₁
- 120: Flüssiger Ablauf aus dem Abtriebsteil der K₁
- 121: Dampf-Flüssigkeitsgemisch aus Sumpfverdampfer der K₁
- 122: Dampfgemisch aus unterem Verstärkungsteil der K₁
- 123: Kondensat des oder der Zwischenkondensatoren der K₁
- 124: Ablauf Flüssigkeitsgemisch aus oberem Verstärkungsteil der K₁
- 125: Rücklauf der K₁
- 126: Restliches Dampfgemisch aus Kondensation der K₁
- 127: Dampfstrom am Kopf der K₁
- 128: Ablauf Flüssigkeitsgemisch aus Reaktionszone oder gegebenenfalls Abtriebsteil der K₂
- 129: Dampf-Flüssigkeitsgemisch aus Sumpfverdampfer der K₂
- 130: Rücklauf der K₂
- 138: Strom enthaltend mittelsiedende Nebenverbindungen
- 150: Strom enthaltend aromatische Hydroxyverbindung und Katalysator
- 151: Strom enthaltend Diarylcarbonat mit hoher Reinheit
- 152: Strom enthaltend schwersiedende Nebenverbindungen und Katalysator
- 153: Strom enthaltend mittelsiedende Nebenverbindungen
- 154: Strom enthaltend schwersiedende Nebenverbindungen und Katalysator zur Entsorgung
- 155: Strom enthaltend schwersiedende Nebnverbindungen und Katalysator
- 156: Brüdenstrom enthaltend Diarylcarbonat zur K₃
- 157: Brüdenstrom enthaltend Diarylcarbonat zur K₄
- 158: Brüdenstrom der K₃
- 159: Restlicher Dampfstrom nach Kondensation in K₃C_{1-N}
- 160: Rücklauf der K₃
- 161: Sumpfprodukt der K₄
- 162: Brüdenstrom der K₄
- 163: Rücklauf der K₄
- 164: Ablauf Flüssigkeit aus dem unteren Abtriebsteil der K₃
- 166: Restbrüdenstrom nach Kondensation in K₄C_{1-N}
- 167: Purgestrom aus dem Sumpfprodukt der K₄
- 169: Brüdenstrom aus Abtriebsteil der K₃ zur Zulaufseite (Ausführung als Trennwandkolonne)

Die folgenden Beispiele dienen der beispielhaften Erläuterung der Erfindung und sind nicht als Beschränkung aufzufassen.

### Beispiele

Die Beispiele sollen das erfindungsgemäße Verfahren anhand der Rückführung des bei der Herstellung von Dimethylcarbonat anfallenden Ethylenglykols zu Ethylencarbonat, dessen Umsetzung mit Methanol zu Dimethylcarbonat, das in das Diphenylcarbonat-Herstellungsverfahren (DPC-Verfahren) mit Phenol eingesetzt werden kann unter Rückführung des bei der Herstellung von Polycarbonat gebildeten Phenols in die Herstellung von Diphenylcarbonat (Fig. 1), darstellen.

### Beispiel

### 1a) Oxidative Carbonylierung von Ethylenglykol mit Kohlenmonoxid und Sauerstoff zu Ethylencarbonat

Ein Autoklav von 20 Vol.-Teilen Größe wurde mit Ethan-1,2-diol (80,33 Gew.-%), PdAc₂ (0,23 Gew.-%), Mn(acac)₃ (7,22 Gew.-%), KBr (12,22 Gew.-%) und DME (2 Vol.-Teile) beschickt. Es wurde mit 20 bar einer Gasmischung aus Stickstoff/Sauerstoff/Kohlenmonoxid (Gewichtsverhältnis 91:3:6) aufgepresst und die Reaktionsmischung für 20 Stunden auf 60°C geheizt. Nach Abkühlen wurde die Reaktionsmischung mit Gaschromatographie analysiert.

Ethylencarbonat wurde in 20,3% Ausbeute erhalten. Dies entspricht einer TON in Bezug auf Palladium von 255 und einer TOF in Bezug auf Palladium von 12,8 h⁻¹. Ethylencarbonat war das einzige detektierbare Produkt.

### 1b) Oxidative Carbonylierung von Propylenglykol mit Kohlenmonoxid und Sauerstoff zu Propylencarbonat

Ein Autoklav von 20 Vol.-Teilen Größe wurde mit Propan-1,2-diol (83,35 Gew.-%), PdAc₂ (0,19 Gew.-%), Mn(acac)₃ (6,11 Gew.-%), KBr (10,35 Gew.-%) und DME (2 Vol.-Teile) beschickt. Es wurde mit 20 bar einer Gasmischung aus Stickstoff/Sauerstoff/Kohlenmonoxid (Gewichtsverhältnis 91:3:6) aufgepresst und die Reaktionsmischung für 20 Stunden auf 60°C geheizt. Nach Abkühlen wurde die Reaktionsmischung mit Gaschromatographie analysiert.

Propylencarbonat wurde in 27,3% Ausbeute erhalten. Dies entspricht einer TON in Bezug auf Palladium von 342 und einer TOF in Bezug auf Palladium von 17,1 h⁻¹. Propylencarbonat war das einzige detektierbare Produkt.

### 2) Umesterung von Ethylencarbonat mit Methanol zu Dimethylcarbonat und Ethylenglykol

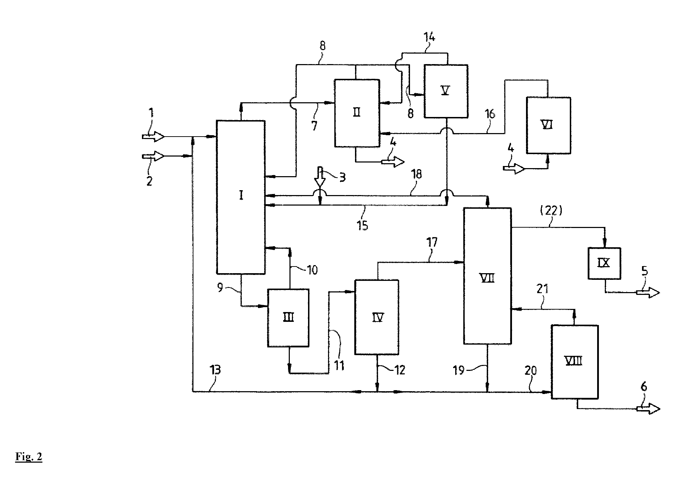

In einer Apparateanordnung gemäß Fig. 2 wurde eine Gegenstromumesterungskolonne (I) mit Abtriebsteil und Verstärkerteil unter Anlegung eines Temperaturgradienten so temperiert, daß die Sumpftemperatur bei etwa 120°C und die Kopftemperatur bei etwa 50°C lag. Über (I) dosierte man 367 Gewichtsteile pro Stunde Ethylencarbonat. Über (8) dosierte man 872 Gewichtsteile pro Stunde eines Gemisches aus 80 Gew.-% Methanol und 20 Gew.-% Dimethylcarbonat. In den unteren Teil der Kolonne, jedoch oberhalb des Abtriebsteils dosierte man über (3) 270 Gewichtsteile pro Stunde Methanol und über (15) weitere 130 Gewichtsteile pro Stunde Methanol. Weiterhin wurden auf den Kopf von (I) über (13) 37 bis 38 Gewichtsteile pro Stunde eines rückgeführten, 4 Gew.-% Katalysator (KOH) enthaltenden Sumpfes und 1,2 Gewichtsteile pro Stunde frisches Kaliumhydroxid über (2) dosiert. Soweit diese eindosierten Stoffströme interne Stoffströme darstellten, wurden sie den in Fig. 2 gezeigten Apparaten entnommen. Die Methanol enthaltenden eindosierten Stoffströme stiegen dampfförmig in der Kolonne nach oben, dem herabfließenden flüssigen und den Katalysator enthaltenden Ethylencarbonat entgegen, wobei die Umesterung zu Dimethylcarbonat und Ethylenglykol erfolgte. Am Kopf von (I) entnahm man 1380 Gewichtsteile pro Stunde eines Gemisches (7) aus 60 Gew.- % Methanol und 40 Gew.-% Dimethylcarbonat, welches einer Bodenkolonne (11) in der Mitte aufgegeben und bei einem Druck von 10 bar in ein Gemisch aus etwa 80 Gew.-% Methanol und 20 Gew.-% Dimethylcarbonat und 378 Gewichtsteile pro Stunde Dimethylcarbonat (4) aufgetrennt wurde. Vom dampfförmig entnommenen Gemisch (8) wurden die genannten 872 Gewichtsteile pro Stunde nach (I) zurückgeführt, während der restliche Teil von (8) in der Kolonne (V) weiter aufgetrennt wurde; hierbei erhielt man ein Dimethylcarbonat enthaltendes Kopfprodukt, welches nach (11) zurückgeführt wurde und 130 Gewichtsteile pro Stunde im wesentlichen aus Methanol bestehendes Sumpfprodukt (15), welches nach (I) zurückgeführt wurde. Das Sumpfprodukt (9) von (I), das im wesentlichen aus Ethylenglykol, geringen Mengen Leichtsiedern (Methanol und Dimethylcarbonat), Hochsiedern, wie Diethylenglykol und dem Katalysator bestand, gelangte in einen Fallfilmverdampfer (III), aus welchem 38 Gewichtsteile pro Stunde Leichtsieder (10) nach (I) zurückgeführt wurden. 375 Gewichtsteile pro Stunde Sumpfprodukt (11) gelangten in einen weiteren Fallfilmverdampfer (IV), dem über seinen Sumpf 75 Gewichtsteile pro Stunde aufkonzentrierte Katalysatorlösung (12) entnommen wurde, die etwa zur Hälfte über (13) nach (I) zurückgeführt und zur anderen Hälfte in einen Dünnschichtverdampfer mit Trennaufsatz (VIII) eindosiert wurde. 302 Gewichtsteile pro Stunde Dampfphase (17) aus (IV) wurden in die Kolonne (VII) eindosiert. In (VII) wurden nochmals 77 Gewichtsteile pro Stunde Leichtsieder (18) abgetrennt, die nach (I) zurückgeführt wurden. Aus dem oberen Teil von (VII) wurden im Seitenabzug 255 bis 256 Gewichtsteile Glykol (22) entnommen, die je nach Reinheitsanforderungen in (IX) weiter auf hochreines Glykol (5) mit einer Menge von 255 Gewichtsteile pro Stunde weiter behandelt werden konnten. Der Sumpfablauf (19) von (VII) in einer Menge von 58 bis 59 Gewichtsteile pro Stunde wurde gemeinsam mit der Hälfte des Sumpfablaufs (12) von (IV) einem Dünnschichtverdampfer (VIII) zugeführt, dessen Destillat (21) mit einer Menge von 91 Gewichtsteile pro Stunde in den unteren Teil von (VII) eingespeist wurde. Der aufkonzentrierte Sumpf (6) von (VIII) mit allen Hochsiedern und einem Teil des Katalysators wurde der Entsorgung zugeführt.

### 3) Herstellung von Diphenylcarbonat durch Umesterung von Dimethylcarbonat mit Phenol

In einer Apparateanordnung gemäß Fig. 3 werden in eine erste Reaktionskolonne (K₁) enthaltend einen oberen Verstärkungsteil (K₁VT₂) mit 4 theoretischen Stufen, einen Zwischenkondensator (K₁IC₁), einen unteren Verstärkungsteil (K₁VT₁) mit 4 theoretischen Stufen, eine Reaktionszone (K₁RZ) mit 30 Reaktionsböden (Hold-up: 12 Volumenteile), wobei 3 Böden mit Heizelementen (K₁E_RZ₁₋₃) ausgestattet sind und einen Abtriebsteil K₁AT mit 6 Böden (Hold-up: 12 Volumenteile) 399,3 Gew.-Teile/h eines Gemisches (118) aus 85,4 Gew.-% Phenol, 9,2 Gew.-% Dimethylcarbonat, 3,2 Gew.-% Diphenylcarbonat, 1,5 Gew.-% Titantetraphenolat, 0,3 Gew.-% Anisol, 0,3 Gew.-% Methylphenylcarbonat und 0,1 Gew.-% Methanol am oberen Ende der Reaktionszone zudosiert. Am unteren Ende der Reaktionszone (K₁RZ) werden 539,6 Gew.-Teile/h eines um 5 °C überhitzten Dampfgemisches (117) aus 98,8 Gew.-% Dimethylcarbonat, 0,9 Gew.-% Phenol, 0,2 Gew.-% Anisol und 0,1 Gew.-% Methanol zugeführt.

Dabei erhält man am Sumpf der Kolonne 452.4 Gew.-Teile/h eines Produktgemisches (106) aus 49.8 Gew.-% Phenol, 28.2 Gew.-% MPC, 12.3 Gew.-% DPC, 8.1 Gew.-% DMC, 0.2 Gew.-% Anisol und 1.4 Gew.-% Titantetraphenolat.

Die K₁ wird bei einem Kopfdruck (oberhalb von K₁VT₂) von 3.6 bar und einem Rücklaufverhältnis von 1.15 betrieben. Dabei wird im Sumpf der Kolonne eine Temperatur von 230 °C und in der Reaktionszone (K₁RZ) eine mittlere Reaktionstemperatur von 215 °C eingestellt. Ein Sumpfverdampfer K₁E₁ und drei Zwischenverdampfer (K₁E_RZ₁₋₃) in der Reaktionszone werden mit Heizdampf bei einem Dampfdruck von 35 bar betrieben, wobei als Sumpfverdampfer (K₁E₁) ein Naturumlaufverdampfer verwendet wird und als Zwischenverdampfer auf den Reaktionsböden integrierte Heizregister verwendet werden. Die Eintrittstemperatur in den Zwischenkondensator (oberhalb von K₁IC₁) beträgt 205°C, die Austrittstemperatur 193 °C und die Kühlleistung 57 kW. Die erforderliche Heizleistung zur Verdampfung des Dimethylcarbonat enthaltenden Stromes (115) beträgt 52 kW. Die Verdampfung und Überhitzung des Dimethylcarbonates erfolgt bei einer Temperatur von 135 bis 152 °C.

Das Sumpfprodukt (106) der ersten Reaktionskolonne (K₁) wird einer zweiten Reaktionskolonne (K₂), enthaltend einen Verstärkungsteil (K₂VT) mit 10 theoretischen Stufen und einen Abtriebsteil (K₂AT) mit 22 theoretischen Stufen zugeführt.

Zusätzlich werden 81,9 Gew.-Teile/h eines Gemisches (109) aus 69,9 Gew.-% Methylphenylcarbonat, 28,3 Gew.-% Phenol, 1.2 Gew.-% Dimethylcarbonat, 0,5 Gew.-% Diphenylether und 0,1 Gew.-% Diphenylcarbonat, aus der Diphenylcarbonat-Feindestillation (K₃) im Bereich des Abtriebsteils (K₂AT) zudosiert.

Dabei werden am Sumpf der zweiten Reaktionskolonne (K₂) 236,6 Gew.-Teile/h eines Produktgemisches (108) aus 62,8 Gew.-% Diphenylcarbonat, 24,2 Gew.-% Methylphenylcarbonat, 9,8 Gew.-% Phenol, 0,4 Gew.-% DMC, 2,6 Gew.-% Titantetraphenolat und 0,2 Gew.-% Diphenylether erhalten.

Ferner werden 238,2 Gew.-Teile/h flüssiges Destillat (103) mit 83,5 Gew.-% Phenol, 15,5 Gew.-% Dimethylcarbonat, 0,6 Gew.-% Methylphenylcarbonat, 0,3 Gew.-% Anisol und 0,1 Gew.-% Methanol entnommen.

Die zweite Reaktionskolonne (K₂) wird bei einem Kopfdruck (oberhalb von K₂VT) von 1 bar und einem Rücklaufverhältnis von 0.65 betrieben. Das aus Abtriebsteil (K₂AT) ablaufende Gemisch hat eine Temperatur von 198 °C und wird in einer zweistufigen Verdampfung zugeführt. Die Austrittstemperatur nach der ersten Verdampfungsstufe (K₂E₁) beträgt 209 °C und nach der zweiten Verdampferstufe (K₂E₂) 230 °C. Als Verdampfer werden in der ersten Stufe ein Naturumlaufverdampfer und in der zweiten Stufe ein Kettle-type-Verdampfer verwendet. Da der Katalysator nicht flüchtig ist, beschränkt sich die Reaktion auf den Abtriebsteil, den Kolonnensumpf und die Verdampfer. Aufgrund der vergleichsweise geringen Temperaturen im Abtriebsteil (188 -198 °C) findet die Reaktion vorwiegend im Kolonnensumpf und den Verdampfern statt.

Das in der zweiten Reaktionskolonne (K₂) erhaltene Sumpfgemisch (108) mit 62,7/24,2/9,8/0,4/2,6/0,03 Gew.-% DPC/MPC/Phenol/DMC/Ti(PhO)₄/Salol und einer Gesamtmenge von 236,6 Gew.-Teile/h wird einer destillativen Aufarbeitung zwecks Isolierung des Diphenylcarbonat, Abtrennung von Hochsiedern und Katalysator und leichtsiedenden Verbindungen zugeführt. Diese besteht aus einer Diphenylcarbonat-Feindestillationskolonne K₃ und einer Diphenylcarbonat-Seitenstromkolonne K₄ gemäß Fig. 4 aufgearbeitet.

Die Diphenylcarbonat-Feindestillationskolonne (K₃) besteht aus vier Sektionen, einem oberen Verstärkungsteil (K₃VT₁) mit 5, einem unteren Verstärkungsteil (K₃VT₂) mit 3, einem oberen Abtriebsteil (K₃AT₁) mit 16 und einem unteren Abtriebsteil (K₃AT₂) mit 9 theoretischen Stufen. Die Kondensation der am Kopf der Kolonne austretenden Dämpfe im Kopfkondensator (K₃C₁) und die teilweise Verdampfung der aus dem unteren Abtriebsteil (K₃AT₂) ablaufenden Flüssigkeit im Verdampfer (K₃E₁) für das Sumpfprodukt erfolgt jeweils einstufig.

Die Diphenylcarbonat-Feindestillationskolonne (K₃) wird bei einem Kopfdruck von 15 mbar und einem Rücklaufverhältnis von 0,7 betrieben.

Dabei erhält man als Destillat (109) einen Strom mit 69,9/28,3/1,2/0,5 Gew.-% MPC/Phenol/DMC/DPE. Unterhalb des oberen Verstärkungsteils (K₃VT₁) werden 0,02 Gew.-Teile/h Flüssigkeit zwecks Ausschleusung von Zwischensiedern im Seitenstrom (153) entnommen. Ferner werden unterhalb des oberen Verstärkungsteils (K₃VT₁) 201 Gew.-Teile/h eines dampfförmigen Seitenstroms (157) mit 99,9 Gew.-% DPC entnommen. Als Sumpfprodukt (155) erhält man 20,6 Gew.-Teile/h eines Gemisches mit 70/29,8/0,2 Gew.-% DPC/Ti(PhO)₄/Salol.

Der dampfförmige Seitenstrom (157) wird einer Seitenstromkolonne (K₄) zugeführt. Diese verfügt lediglich über einen Verstärkungsteil (K₄VT) mit 9 theoretischen Stufen.

Die Seitenstromkolonne (K₄) wird unter gleichen Druckbedingungen wie die DPC-Feindestillationskolonne (K₃) und bei einem Rücklaufverhältnis von 0,5 betrieben.

Die am Kopf der Seitenstromkolonne (K₄) austretenden Dämpfe (162) werden in einer zweistufigen Kondensation in den Kondensatoren (K₄C₁₋₂)kondensiert, wobei die Kondensationswärme entweder zur Erzeugung von Dampf oder zur Erwärmung anderer Prozessabschnitte der DPC-Herstellung verwendet wird.

Dabei erhält man ein Destillat (151) mit 99,96 Gew.-% DPC und nur 300 ppm Salol. Die am Sumpf der Seitenstromkolonne ablaufende Flüssigkeit (161) wird der Diphenylcarbonat-Feindestillationskolonne (K₃) oberhalb des unteren Abtriebsteils (K₃AT₂) zugeführt.

### 4) Herstellung von Polycarbonat aus Bisphenol A und Diphenylcarbonat

Aus einer Vorlage werden 8,600 Gew.-Teile/h Schmelzegemisch, bestehend aus 4.425 Gew.-Teilen/h Diphenylcarbonat, hergestellt wie beschrieben in Beispiel 4), und 4,175 Gew.-Teilen/h Bisphenol A, unter Zusetzen von 0,52 Gew.-Teilen/h des Phenoladdukts von Tetraphenylphosphoniumphenolat mit 65,5 % Tetraphenylphosphoniumphenolat/h gelöst in 4,5 Gew.-Teile/h Phenol/h durch einen Wärmetauscher gepumpt, auf 190°C erwärmt und durch eine Verweilkolonne bei 12 bar und 190°C geführt. Die mittlere Verweilzeit beträgt 50 Minuten.

Die Schmelze wird dann über ein Entspannungsventil in einen unter 200 mbar stehenden Abscheider geleitet. Die abfließende Schmelze wird in einem, ebenfalls unter 200 mbar stehenden, Fallfilmverdampfer wieder auf 189°C erwärmt und in einer Vorlage aufgefangen. Nach einer Verweilzeit von 20 Minuten wird die Schmelze in die nächsten drei, gleichartig aufgebauten Stufen gepumpt. Die Bedingungen in der 2. / 3. / 4. Stufe sind 100 / 74 / 40 mbar, 218 / 251 / 276°C und 20 / 10 / 10 Minuten. Das entstandene Oligomere hat eine relative Viskosität von 1,09. Alle Brüden werden über Druckregelungen in eine unter Vakuum stehende Kolonne geführt und als Kondensate abgeleitet.

Danach wird das Oligomer in einem sich anschließenden Korbreaktor bei 278°C und 3,0 mbar bei einer Verweilzeit von 45 Minuten zu einem höhermolekularen Produkt aufkondensiert. Die relative Viskosität beträgt 1,195. Die Brüden werden kondensiert.

Vom Schmelzestrom, der in einen weiteren Korbreaktor geleitet wird, wird mittels einer Zahnradpumpe ein Teilstrom von 150 Gew.-Teilen/h Schmelze abgezweigt, mit 0,185 Gew.-Teilen/h einer 5 %-igen wässrigen Phosphorsäure versetzt, über einen statischen Mischer mit einem Länge-zu-Durchmesser-Verhältnis von 20 gerührt und wieder in den Hautschmelzestrom zurückgeleitet. Direkt nach dem Zusammentreffen wird die Phosphorsäure im gesamten Schmelzestrom mittels eines weiteren statischen Mischers homogen verteilt.

Die so behandelte Schmelze wird in einem weiteren Korbreaktor bei 284°C, 0,7 mbar und bei einer mittleren Verweilzeit von 130 Minuten weiter den Prozessbedingungen ausgesetzt, ausgetragen und granuliert.

Die Brüden werden in der Vakuumanlage und dahinter kondensiert.

Das erhaltene Polycarbonat hat folgende Kennzahlen: relative Viskosität 1,201 / phenolische OH 255 [ppm] / DPC 71 [ppm] / BPA 6 [ppm] / Phenol 56 [ppm].

Das abdestillierte Phenol kann wieder in die Diphenylcarbonatherstellung gemäß Schritt (g), wie beschrieben in Beispiel 4), rückgeführt werden.

## Patentansprüche

1. Verfahren zur Herstellung von wenigstens einem Diarylcarbonat aus wenigstens einem Dialkylcarbonat und wenigstens einer aromatischen Hydroxyverbindung, umfassend folgende Schritte:
(a) Herstellung von Alkylencarbonat durch oxidative Carbonylierung von Alkylenglycol mit Kohlenmonoxid und Sauerstoff unter Bildung von Wasser,
(b) Abtrennung und gegebenenfalls Reinigung wenigstens eines Teils des in Schritt (a) gebildeten Alkylencarbonats,
(c) Umsetzung des gemäß Schritt (b) abgetrennten Alkylencarbonats mit wenigstens einem Alkylalkohol in Gegenwart eines Katalysators, und gegebenenfalls organischem Lösungsmittel unter Bildung wenigstens eines Dialkylcarbonats und von Alkylenglykol,
(d) Abtrennung und Aufarbeitung wenigstens eines Teils des in Schritt (c) gebildeten Dialkylcarbonats,
(e) Abtrennung und gegebenenfalls Reinigung wenigstens eines Teils des gemäß Schritt (c) gebildeten Alkylenglykols,
(f) Rückführung wenigstens eines Teils des gemäß Schritt (e) abgetrennten Alkylenglykols, gegebenenfalls enthaltend Dialkylcarbonat(e), zum Schritt (a),
(g) Umsetzung wenigstens eines Teils des gemäß Schritt (e) abgetrennten Dialkylcarbonats mit einem Monophenol zum Alkylarylcarbonat und/oder Diarylcarbonat und Alkylalkohol; das Alkylalkohol kann optional wiederum in Schritt (c) eingesetzt werden,
(h) Disproportionierung wenigstens eines Teils des gemäß Schritt (g) hergestellten Alkylarylcarbonats zum Diarylcarbonat und Dialkylcarbonat, das wiederum in Schritt (c) eingesetzt werden kann,
(i) Umesterung wenigstens eines Teils des gemäß Schritt (h) hergestellten Diarylcarbonats mit einem Bisphenol zum Oligo-/Polycarbonat und dem Monophenol; das Monophenol kann wiederum in Schritt (g) eingesetzt werden.

2. Verfahren gemäß Anspruch 1, **dadurch gekennzeichnet, dass** das zurückgeführte Alkylenglykol aus Schritt (f) zum Schritt (a) weniger oder gleich 1000 mol ppm Dialkylcarbonat(e) enthält.

3. Verfahren gemäß Anspruch 1 oder 2, **dadurch gekennzeichnet, dass** die Aufreinigung des Alkylenglykols destillativ erfolgt.

4. Verfahren gemäß einem der Ansprüche 1 bis 3, **dadurch gekennzeichnet, dass** das aus dem Gemisch in (b) abgetrennte Alkylenglykol gemeinsam mit dem rückgeführten Strom aus (f) zurück in den Schritt (a) gelangt.

5. Verfahren gemäß Anspruch 1, **dadurch gekennzeichnet, dass** die oxidative Carbonylierung in Schritt (a) als Katalysator wenigstens ein Edelmetall, ausgewählt aus Palladium, Rhodium, Iridium und Platin in der elementaren Form oder als ihre ionischen oder nicht-ionischen Verbindungen und als Cokatalysator wenigstens eine Verbindung ausgewählt aus Mangan-, Kobalt- oder Kupferverbindungen

6. Verfahren gemäß Anspruch 2, **dadurch gekennzeichnet, dass** als Katalysator Salze oder Organometallische Verbindungen von Palladium in der Oxidationsstufe II, Rhodium in der Oxidationsstufe I oder III, Iridium in der Oxidationsstufe I oder III oder Platin in der Oxidationsstufe II verwendet werden.

7. Verfahren gemäß Anspruch 2 oder 3, **dadurch gekennzeichnet, dass** als Katalysator Palladium in elementarer Form oder als ionische oder nicht ionische Verbindung verwendet wird.

8. Verfahren gemäß einem der Ansprüche 2 bis 4, **dadurch gekennzeichnet, dass** als redoxaktiver Cokatalysator wenigstens eine Verbindung ausgewählt aus Mangan-, Kobalt- oder Kupferverbindungen, in einem Gewichtsverhältnis Katalysatorverbindung zu redoxaktiver Substanz von 1:1 bis 1:100, vorzugsweise 1:2 bis 1:30 eingesetzt wird.

9. Verfahren gemäß einem der Ansprüche 1 bis 5, **dadurch gekennzeichnet, dass** die zyklischen Alkylencarbonate solche mit der Formel (I) sind: 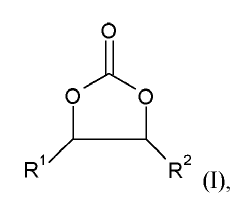 wobei in der Formel R¹ und R² unabhängig voneinander Wasserstoff, substituiertes oder nicht substituiertes **C₁-C₄-Alkyl**, substituiertes oder nicht substituiertes **C₂-C₄-Alkenyl** oder substituiertes oder nicht substituiertes **C₆-C₁₂-Aryl** und R³ und R⁴ gemeinsam mit den beiden Fünfring-C-Atomen einen gesättigten carbozyklischen Ring mit 5 - 8 Ringgliedern bedeuten können.

10. Verfahren gemäß Anspruch 6, **dadurch gekennzeichnet, dass** das zyklische Alkylencarbonat Ethylencarbonat, Propylencarbonat oder Butylencarbonat ist.

11. Verfahren gemäß einem der Ansprüche 1 bis 7, **dadurch gekennzeichnet, dass** Dialkylcarbonate der Formel (II) verwendet werden: 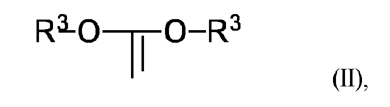 R³ steht für lineares oder verzweigtes, gegebenenfalls substituiertes C₁-C₃₄-Alkyl, bevorzugt für C₁-C₆-Alkyl, besonders bevorzugt für C₁-C₄-Alkyl.

12. Verfahren gemäß Anspruch 8, **dadurch gekennzeichnet, dass** das Dialkylcarbonat Dimethylcarbonat, Diethylcarbonat, Di(n-propyl)carbonat, Di(iso-propyl)carbonat, Di(n-butyl)carbonat, Di(sec-butyl)carbonat, Di(tert-butyl)carbonat oder Dihexylcarbonat ist.

13. Verfahren gemäß einem der Ansprüche 1 bis 9, **dadurch gekennzeichnet, dass** als Diarylcarbonate solche der allgemeinen Formel (VII) verwendet werden: 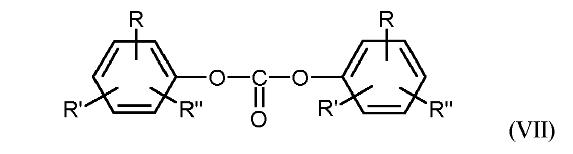 wobei R, R' und R" unabhängig voneinander H, lineares oder verzweigtes, gegebenenfalls substituiertes C₁-C₃₄-Alkyl, C₁-C₃₄-Alkoxy, C₅-C₃₄-Cycloalkyl, C₇-C₃₄-Alkylaryl, C₆-C₃₄-Aryl oder einen Halogenrest darstellen und R, R' und R" auf beiden Seiten der Formel (VII) gleich oder verschieden sein können. R kann auch -COO-R"' bedeuten, wobei R"' H, gegebenenfalls verzweigtes C₁-C₃₄ Alkyl, C₁-C₃₄-Alkoxy, C₅-C₃₄-Cycloalkyl, C₇-C₃₄-Alkylaryl oder C₆-C₃₄-Aryl sein kann.

14. Verfahren gemäß Anspruch 10, **dadurch gekennzeichnet, dass** das Diarylcarbonat Diphenylcarbonat, 4-tert-Butylphenyl-phenyl-carbonat, Di-(4-tert-butylphenyl)-carbonat, Biphenyl-4-yl-phenyl-carbonat, Di-(biphenyl-4-yl)-carbonat, 4-(1-Methyl-1-phenylethyl)-phenyl-phenyl-carbonat und Di-[4-(1-methyl-1-phenylethyl)-phenyl]-carbonat ist.

15. Verfahren zur Herstellung von Polycarbonat, wobei das Diarylcarbonat gemäß einem der Ansprüche 1 bis 14 mit Bisphenol in der Schmelze umgesetzt wird und Polycarbonat und Monophenol entstehen.

16. Verfahren zur Herstellung von Polycarbonat gemäß Anspruch 15 **dadurch gekennzeichnet, dass** das Monophenol aus der Polycarbonatherstellung in Schritt (g) zurückgeführt wird.

17. Polycarbonat erhältlich aus den Verfahren gemäß einem der Ansprüche 15 bis 16.
